# EUROPÄISCHE PATENTANMELDUNG

(11) **EP 1 600 504 A1**
(43) Veröffentlichungstag der Anmeldung: **30.11.2005**
(21) Anmeldenummer: 04012758.1
(22) Anmeldetag: 28.05.2004
(51) Int. Cl.: C12N 9/10, C07K 5/00

(54) **Steuerung der Transglutaminase-katalysierten Proteinmodifizierung mittels selektiver irreversibler Transglutaminase-Inhibitoren**

(71) Anmelder: N-Zyme BioTec GmbH, 64295 Darmstadt (DE)
(72) Erfinder: Pasternack, Ralf, 64347 Griesheim (DE); Oertel, Kai, 55122 Mainz (DE); Otterbach Noä, Jens, 64347 Griesheim (DE); Raile, Renate, 64367 Mühltal (DE)
(74) Vertreter: Grünecker, Kinkeldey, Stockmair & Schwanhäusser Anwaltssozietät

(57) **Zusammenfassung**

Die vorliegende Erfindung betrifft ein Verfahren zur Transglutaminase-katalysierten Proteinmodifizierung, welches durch selektive irreversible Transglutaminase-Inhibitoren gesteuert wird, sowie neue Inhibitoren der verwendeten Transglutaminasen und Verfahren zu deren Herstellung.

## Beschreibung

Die vorliegende Erfindung betrifft ein Verfahren zur Transglutaminase-katalysierten Proteinmodifizierung, welches durch selektive irreversible Transglutaminase-Inhibitoren gesteuert wird, sowie neue Inhibitoren der verwendeten Transglutaminasen und Verfahren zu deren Herstellung.

Eine sehr häufig praktizierte Art der Proteinmodifizierung ist die Markierung von Proteinen mit Markersubstanzen. Eine Übersicht über die gängigsten Methoden findet sich beispielsweise bei Lundblad R.L., *Techniques in Proteinmodification, CRC Press,* 1995, sowie Hermanson G.T., *Bioconjugate Techniques, Academic Press,* 1996. Diese Art der Modifizierung von Proteinen besitzt in der biologischen, medizinischen und pharmazeutischen Forschung hohe Relevanz, da die Aufklärung von molekularen Vorgängen, an denen diese Proteine beteiligt sind, erst durch ihre Markierung möglich wird. Auch diagnostische Verfahren arbeiten mit solchen markierten Proteinen. Das Einbringen der Markermoleküle wird dabei über reaktive Gruppen realisiert, die mit funktionellen Gruppen am Protein reagieren. Die Durchführung solcher Modifikationen ist ungeachtet ihrer breiten Anwendung in Industrie und Forschung nicht trivial, wenn ein hochwertiges Proteinprodukt hergestellt werden soll. Hochwertig bezieht sich in diesem Fall auf die Ausbeute, die Einstellung eines gewünschten Modifizierungsgrades, die Löslichkeit des Produktes sowie unter Umständen sogar den Erhalt der Funktionalität.

Proteinmodifizierungen, insbesondere Markierungen, werden am häufigsten chemisch durchgeführt. Darunter versteht man die direkte Umsetzung eines Proteins mit einem Reaktionspartner, insbesondere einem Farbstoff oder einem anderen Markermolekül (z. B. mit Biotin, einem Biotinderivat, einem radioaktiven Marker, einem Fluoreszenz- oder Nichtfluoreszenzfarbstoff etc.), der eine funktionelle Gruppe enthält, die mit funktionellen Aminosäuren des Proteins reagieren kann. In vielen Fällen handelt es sich dabei um sogenannte Aktivester von Carbonsäuren, die mit zugänglichen Lysinresten des Proteins reagieren. Zum Einsatz kommen aber auch andere funktionelle Gruppen, wie zum Beispiel Maleinimide oder lodacetamide, die mit freien Cysteinresten des Proteins in Reaktion treten.

In der Regel sind diese chemischen Reaktionen schwer zu steuern, d. h. da ein Protein über eine Vielzahl von beispielsweise Lysinen verfügt, werden sehr inhomogene Reaktionsprodukte erhalten. Die Verteilung von Markermolekülen, welche die Proteinmoleküle nach einer solchen Reaktion tragen, kann von null (unmodifiziertes Protein) bis ca. vierzig Markermolekühlen pro Proteinmolekül reichen. Als direkte Folge dieser Inhomogenität ist es grundsätzlich nicht möglich, die Menge der Proteine in den Anwendungen über das Markersignal direkt zu quantifizieren. Zusätzlich verändern sich die Eigenschaften wie zum Beispiel die Löslichkeit eines Proteins durch die zusätzlichen Gruppen. Bei einer breiten Produktverteilung erhält man so auch ein in seinen molekularen Eigenschaften stark variierendes Produktgemisch.

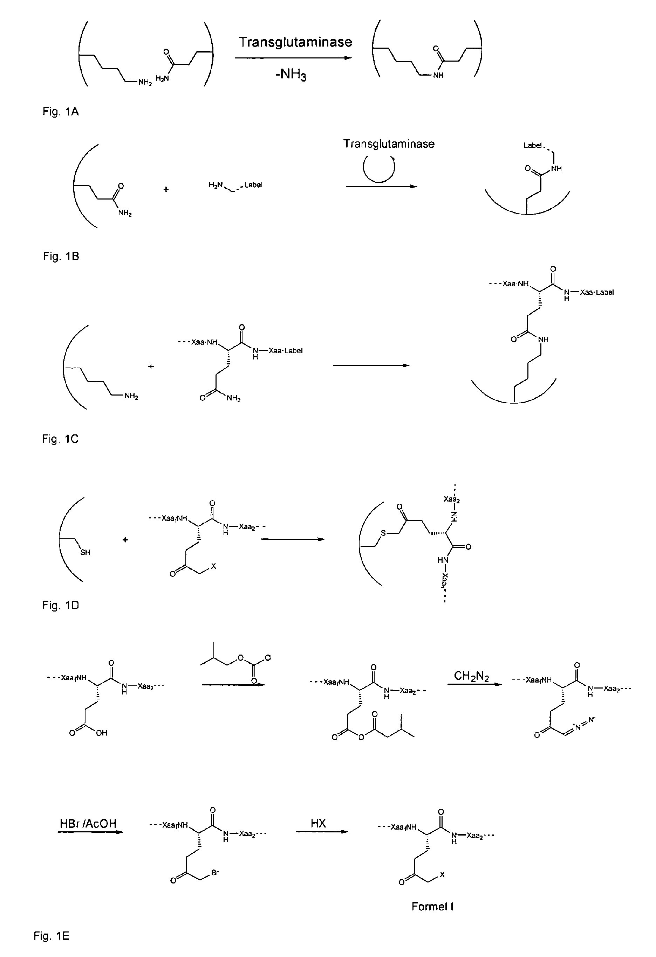

Eine Alternative zu der chemischen Modifizierung bietet die Verwendung von Enzymen, insbesondere die Verwendung von Transglutaminasen. In der Literatur ist seit langem bekannt, dass die biologische Reaktion der Transglutaminasen, Quervernetzung von Proteinen (siehe Fig. 1A), auch genutzt werden kann, um Proteine enzymatisch zu modifizieren. Dabei wird beispielsweise ein Protein, welches zugängliche Glutaminseitenketten aufweist, mit Markermolekülen modifiziert, die ein primäres Amin enthalten (Fig. 1 B). Auch die Darstellung von Konjugaten aus verschiedenen Proteinen unter Transglutaminasekatalyse ist in der Literatur beschrieben (Bechthold U., J. Biochem (Tokio), Feb. 2000; 127(2):239-45).

Nur wenige Publikationen beschäftigen sich mit der umgekehrten Reaktionsführung, d. h. mit der Markierung von Lysinresten unter Verwendung von Peptidderivaten (Fig. 1 C). Pasternack und Mitarbeiter beschreiben den Einbau eines N-terminal geschützten Dipeptidderivates, das mit dem Dansylrest verknüpft ist (Pasternack et al., Anal. Biochem., 1997, 54-60). Verschiedene Autoren beschreiben die Nutzung eines Biotin-tragenden, glutaminhaltigen Hexapetids zum Auffinden von reaktiven Lysinresten in Proteinen (Groenen et al., J. Biol. Chem., 1994, Ruoppolo et al.; Protein Science, 12, 2003, 1290-1297; Orrü et al., J. Biol. Chem., 278, 2003, 31766-31773). Ziel dieser Arbeiten ist es, ein vereinfachtes analytisches Verfahren zur Identifizierung von Substratproteinen in komplexen biologischen Matrices zu erreichen. Die gesteuerte Modifizierung der Proteine ist mit den dort beschriebenen Verfahren nicht möglich. Für industrielle Zwecke ist es jedoch erforderlich, ein steuerbares Verfahren zu entwickeln, um definierte Modifizierungsgrade zu realisieren. Weitere Veröffentlichungen, die in diesem Zusammenhang zu erwähnen sind: Lorand L. et al., Proc. Natl. Acad. Sci USA, 1991, 88, 82-83 sowie Lorand L. et al., Proc. Natl. Acad. Sci USA, 1992, 89, 11161-63.

Weist ein Protein keine zugänglichen Lysin- oder Glutaminreste auf, d. h. ist es in seiner natürlichen Form kein Substrat von Transglutaminasen, so kann durch molekulargenetische Methoden ein geringfügig verändertes Protein hergestellt werden, indem gezielt eine Bindungsstelle für Transglutaminasen eingeführt wird. Das US-Patent 6,322,996 beschreibt ein Verfahren, bei dem ein Zielprotein mit einer Peptid-Sequenz, die ein Glutamin enthält, fusioniert wird. Dadurch wird das Protein zu einem Substrat für Transglutaminase, was nach Angaben der Erfinder eine spezifische Modifizierung ermöglicht. Ein wesentliches Motiv ist es dort, pharmazeutische Proteine durch Aminderivate von Polyethylenglycol zu modifizieren. Zielsetzung ist die Veränderung von Eigenschaften wie der Serumhalbwertszeit und der Löslichkeit des Proteins. In der Patentveröffentlichung EP1151299 wird die Fusion eines Proteins mit der Sequenz Gln-Ser-Lys-Val beschrieben, um Proteine, die in ihrer nativen Form kein zugängliches Glutamin aufweisen, in ein Transglutaminasesubstrat zu überführen. Als Anwendungen werden hier verschiedene Markermoleküle für Proteine genannt, die als gemeinsames Merkmal eine lysinanaloge primäre Aminogruppe enthalten. Weitere Patentschriften, die die Verwendung glutaminhaltiger Proteine und niedermolekularer Amindonoren in transglutaminase-katalysierten Labelingreaktionen beschreiben sind: US 6,010,871 sowie US 6,607,740.

In einer jüngst erschienenen Veröffentlichung weisen die Autoren die besonderen Vorteile der Transglutaminase-katalysierten Markierung eines Proteins nach. Glutathion-S-transferase (GST) wird, nach Expression mit einem N- bzw. C-terminalen Tag, durch Meerschweinchenlebertransglutaminase mit Fluoresceincadaverin markiert. Das so dargestellte Protein wird mit einer chemisch gelabelten GST hinsichtlich seiner Eigenschaften verglichen. Insbesondere die Aktivität der GST ist bei dem durch Transglutaminase markierten Protein deutlich erhöht. (Taki et al., Protein Engineering, Desing & Selection, 2004 17 (2), 119-26.) Eine Steuerung des Prozesses ist in diesem Spezialfall nicht erforderlich, da nur die zusätzlich in das Protein eingebrachten Tag-Sequenzen von der eingesetzten Transglutaminase als Substrat akzeptiert werden. Die Vorteile, die ein selektiv markiertes Protein gegenüber einem nach dem verbreiteten Stand der Technik (chemisches Labeling) markierten Protein hat, werden in dieser Publikation dennoch deutlich.

Die oben genannten Publikationen beschreiben zwar die Durchführung der eigentlichen Transglutaminase-katalysierten Reaktionen, dies geschieht dort jedoch in einem ungesteuerten Prozess. Weist aber ein Protein mehrere Glutamin- oder Lysinfunktionalitäten auf, denen eine Reaktion mit Transglutaminase möglich ist, so erhält man in einem ungesteuerten Prozess in der Regel wiederum ein Gemisch von Produkten mit unterschiedlichem Markierungsgrad.

Ein weiteres ungelöstes Problem der publizierten Verfahren ist der Verbleib aktiver Transglutaminase im fertigen Proteinprodukt. Diese führt zu unerwünschten Folgereaktionen, insbesondere Vernetzung des Proteinprodukts. Eine unspezifische Denaturierung der Transglutaminase (z. B. durch Hitze oder pH-Wert) ist wegen der gleichzeitigen Auswirkungen auf das Zielprotein nicht möglich; eine Abtrennung der Transglutaminase oder eines niedermolekularen Reaktionspartners, z. B. eines Markermoleküls, mit den üblichen Trennungsverfahren kann in der Regel nicht schnell genug erfolgen und beeinträchtigt auch die Ausbeute an Proteinprodukt.

In Anbetracht der geschilderten Nachteile des Standes der Technik, besteht die Aufgabe der vorliegenden Erfindung in der erstmaligen Bereitstellung eines Transglutaminase-katalysierten Proteinmodifizierungsverfahrens, welches zur kontrollierten Herstellung von spezifischen Proteinprodukten geeignet ist.

Diese Aufgabe wird erfindungsgemäß gelöst durch ein Verfahren zur Transglutaminase-katalysierten Proteinmodifizierung, welches durch einen selektiven irreversiblen Transglutaminase-Inhibitor gesteuert wird.

Durch die Verwendung eines solchen Inhibitors kann die katalytische Reaktion sehr schnell und vollständig zu einem gewünschten Zeitpunkt beendet werden, was den Einsatz der bekannten Transglutaminase-katalysierten Reaktion zwischen Proteinen und Peptiden bzw. Aminen zur Herstellung hochspezifisch modifizierter, insbesondere markierter, Proteine erst möglich macht. Da die Reaktionsgeschwindigkeit verschiedener Glutamin- bzw. Lysinreste in einem Protein sich in den meisten Fällen zumindest leicht unterscheidet, kann durch ein schnelles Abstoppen der Reaktion ein weitgehend einheitliches Produkt erhalten werden. Für das Abstoppen der Reaktion werden Transglutaminase-Inhibitoren benötigt, die selektiv und irreversibel mit dem Cystein im Aktivzentrum der Transglutaminasen reagieren. Aufgrund der Inaktivität gegenüber "Nicht-Aktivzentrumscysteinen" ist eine unerwünschte Modifikation des Zielproteins mit dem Inhibitormolekül ausgeschlossen. Der streng irreversible Charakter der Transglutaminase-Inhibition lässt nur vollständig inaktives Enzym zurück. Als weiterer Vorteil der Erfindung wird das Zielprotein somit nicht mit aktivem Fremdprotein kontaminiert.

Für den Einsatz in der vorliegenden Erfindung sind grundsätzlich alle selektiven irreversiblen Transglutaminase-Inhibitoren geeignet, welche die jeweils eingesetzte(n) Transglutaminase(n) hinreichend schnell und vollständig hemmen.

In einer bevorzugten Ausführungsform werden Inhibitoren der folgenden Formel I verwendet. 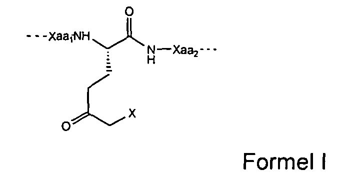 worin X eine Austrittsgruppe für eine nukleophile Substitution, vorzugsweise N₂, S⁺(CH₃)₂, RCOO oder R-S, ist und Xaa₁ und Xaa₂ gleich oder verschieden sein können und jeweils eine Aminoschutzgruppe, eine Aminosäure oder ein Aminosäurederivat oder eine Peptidylgruppierung mit 2-8, vorzugsweise 6-8, Aminosäureresten oder Derivaten von Aminosäuren darstellen.

In der obigen Formel I bedeutet RCOO eine Carboxylgruppe, vorzugsweise eine aromatische Carboxylgruppe, und die resultierenden Inhibitoren werden als Acyloxymethylketone bezeichnet. R-S bedeutet einen Thioether, vorzugsweise einen Thioether, dessen Schwefelatom an eine aromatische oder resonanzstabilisierte Gruppe gebunden ist. Bevorzugte Beispiele solcher Inhibitoren sind Thioimidazoliummethylketone.

Die Aminosäurederivate schließen insbesondere Aminosäuren ein, deren freie primäre Aminogruppe, falls vorhanden, mit einer üblichen Schutzgruppe, besonders bevorzugt tert.-Butyloxycarbonyl oder Benzyloxycarbonyl, geschützt ist oder deren freie Carboxylgruppe, falls vorhanden, mit einem Alkohol, z. B. einem niederen Alkanol, verestert ist. Alternative oder zusätzliche Derivatisierungen der Aminosäuren sind möglich, sofern die inhibitorische Aktivität der resultierenden Verbindungen nicht beeinträchtigt wird.

Die Inhibitoren der obigen Formel I tauschen in einem irreversiblen Reaktionsschritt das Thiolat des Cysteins im aktiven Zentrum der Transglutaminase gegen die jeweilige Austrittsgruppe X aus (siehe Fig. 1D).

Ein allgemeines Verfahren zur Herstellung geeigneter Transglutaminase-Inhibitoren, insbesondere der Formel I, beinhaltet, dass in einem als Transglutaminasesubstrat bekannten Peptid das Substratglutamin durch eine in Gammaposition in ein Methylketon umgewandelte Glutaminsäure ersetzt wird. In einer bevorzugten Ausführungsform geschieht dies durch ein dreistufiges Verfahren ausgehend von einem Peptid, in dem die Gammaposition der Ziel-Glutaminsäure als einzige funktionelle Gruppe ungeschützt vorliegt. Die Aktivierung dieser Carbonsäure erfolgt dabei als Mischanhydrid durch vorzugsweise Isobutylchloroformiat. Durch Zusatz von Diazomethan wird die Carbonsäure in ein Methylketon überführt. Aus diesem Diazomethylketon (X=N₂), welches bereits selbst einen erfindungsgemäßen Inhibitor darstellt, kann durch Umsetzung mit z.B. Bromwasserstoffsäure in Essigsäure ein reaktives Intermediat dargestellt werden (Brommethylketon, X= Br). Substitution des Broms durch Carboxylate, Thiole oder andere Anionen liefert Methylketone unterschiedlicher Aktivität. (in Fig. 1E schematisch dargestellt).

Spezielle Beispiele geeigneter Transglutaminase-Inhibitoren der Formel I sind N°-Benzyloxycarbonyl-L-(6-diazo-5-oxonorleucinyl)glycin (Z-DON-Gly-OH), N^{α}-Benzyloxycarbonyl-L-(6-dimethylsulfonium-5-oxonorleucinyl)glycinmethylester (Z-SON-Gly-OMe), N^{α}-Benzyloxycarbonyl-L-(6-diazo-5-oxonorleucinyl)phenylalaninmethylester (Z-DON-Phe-OMe), N°-Benzyloxycarbonyl-L-(6-dimethylsulfonium-5-oxonorleucinyl)phenylalaninmethylester (Z-SON-Phe-OMe), N^{α}-tert.-Butyloxycarbonyl-L-(6-diazo-5-oxonorleucinyl)-glutaminyl-L-isoleucinyl-L-valinmethylester (Boc-DON-Gln-Ile-Val-OMe), N^{α}-tert.-Butyloxycarbonyl-L-(6-dimethylsulfonium-5-oxonorleucinyl)-L-glutaminyl-L-isoleucinyl-L-valinmethylester (Boc-SON-Gln-Ile-Val-OMe), N^{α}-tert.-Butyloxycarbonyl-L-pyroglutaminyl-L-alaninyl-L-(6-diazo-5-oxonorleucinyl)-L-glutaminyl-L-isoleucinyl-L-valinmethylester (Boc-Pyr-Ala-DON-Gln-Ile-Val-OMe).

Die oben genannten Inhibitoren sind neu und detaillierte Verfahren zu ihrer Herstellung werden im Abschnitt SYNTHESEBEISPIELE beschrieben. Ein weiteres Beispiel eines geeigneten Inhibitors, Ac-Prolinyl-glutaminyl-prolinyl-(6-diazo-5-oxonorleucinyl)-leucinylprolinyl-phenylalaninyl-NH₂, (Ac-ProGlnPro-DON-LeuProPhe-NH₂) wurde bereits in der Literatur (Hausch et al., Chemistry and Biology, Bd. 10, 2003, 225-321) beschrieben und als mögliches Medikament gegen Zöliakie genannt, jedoch nicht zur Steuerung einer Transglutaminase-katalysierten Proteinmodifizierung verwendet oder vorgeschlagen

Die hier beschriebenen Inhibitoren der Formel I wirken irreversibel, da eine kovalente Bindung mit dem Cystein des Aktivzentrums ausgebildet wird (Fig. 1D). Die Inhibitoren sind insoweit selektiv, als die Cysteinmoleküle, die nicht das Aktivzentrum eines Enzyms mit einer Cys-His-Diade bilden, bis zu einem pH-Wert von 8 nicht mit dem Inhibitor reagieren.

Weitere, jedoch nicht beschränkende, Beispiele geeigneter selektiver irreversibler Transglutaminase-Inhibitoren, welche nicht von der Formel I umfasst werden, sind N°-Benzyloxycarbonyl-L-serinyl-(O-chloracetyl)glycin (Z-Ser(OAcCl)-Gly-OH) (de Macedo et al., Bioorg. & Med. Chem., 10, 2002, 355-360), N^{α}-Benzyloxycarbonyl-L-lysinyl(ε-acryloyl)glycin (Z-Lys(Acryloyl)-Gly-OH) (de Macedo et al., Bioorg. & Med. Chem., 10, 2002, 355-360) und N-(N°-Benzyloxycarbonyl-L-phenylalaninyl)-(4-amino-2-oxo-dimethylsulfonium)pentan-Bromidsalz (Z-Phe-NH-(CH₂)₃-CO-S⁺(CH₃)₂Br⁻) (Pliura et al., J. Enzyme Inhibition, 6, 1992, 181-194) und verwandte Verbindungen. Keiner dieser aus der Literatur bekannten Inhibitoren wurde bisher zur Steuerung einer Transglutaminase-katalysierten Proteinmodifizierung verwendet oder vorgeschlagen.

Im Prinzip kann jede Transglutaminase für das beschriebene Verfahren genutzt werden, denn alle Mitglieder der Enzymfamilie katalysieren grundsätzlich dieselbe Reaktion. Derzeit bevorzugt sind bakterielle Transglutaminasen und Transglutaminasen aus Vertebraten, insbesondere des Menschen. Im Moment sind 9 Transglutaminasen beim Menschen beschrieben. Homologe der bekannten Transglutaminasen könnten nach bekannten Verfahren aus anderen Spezies, z.B. aus Pilzen und Hefe, isoliert werden. Im vorliegenden Patent werden beispielhaft Inhibitoren für rekombinante humane Transglutaminase 2, Meerschweinchenleber-Transglutaminase, humanen Faktor XIIIa und mikrobielle Transglutaminase aus *streptomyces mobaraensis* näher beschrieben und getestet.

In einer speziellen Ausführungsform des erfindungsgemäßen Verfahrens werden mehrere Transglutaminasen mit unterschiedlicher Substratspezifität verwendet. Diese Transglutaminasen können nacheinander oder gleichzeitig eingesetzt werden. Bei der Verwendung mehrerer Transglutaminasen können diese gleichzeitig oder in zeitlich abgestimmter Reihenfolge inhibiert werden. Auf diese Weise können z.B. zwei unterschiedliche Label oder andere Modifizierungen selektiv eingebracht werden. Das Zusammenwirken der divergenten Substratspezifität verschiedener Transglutaminasen und der Möglichkeit, jede dieser Transglutaminasen schnell und vollständig zu inhibieren, ermöglicht die routinemäßige Produktion hochwertig modifizierter Proteine mit einem genau einstellbaren Markierungs- bzw Modifizierungsgrad in einem geregelten technischen Prozess.

In einem speziellen Aspekt der vorliegenden Erfindung ist das Verfahren zur Proteinmodifizierung ein Markierungsverfahren. Wie bereits in der Einleitung angesprochen, wird das Protein dabei mit einem Markermolekül umgesetzt, das neben der nachweisbaren Funktion eine weitere funktionelle Gruppe enthält, die mit einer funktionellen Aminosäure des Proteins reagieren kann. Nachdem das erfindungsgemäße Verfahren Transglutaminase-katalysiert ist, muss das Markermolekül ein Substrat für eine Transglutaminase sein und entweder eine reaktionsfähige primäre Aminogruppe oder einen Glutaminrest enthalten. Bei den Markermolekülen kann es sich z. B. um Peptide handeln, die ein oder mehrere Lysine enthalten, um niedermolekulare Markermoleküle, die mit einem primären Amin (häufig Cadaverin) versehen sind, um Makromoleküle, die ein geeignetes primäres Amin enthalten, oder sogar um monoklonale Antikörper.

Die nachweisbare Marker-Funktion kann z. B. ein Farbstoff, Fluoreszenzfarbstoff, Chemolumineszenzfarbstoff, Enzymlabel, Redoxlabel, Immunlabel, Biotin-Label, Isotopenlabel, Spinlabel oder irgendeine andere bekannte Marker-Funktion sein.

Geeignete Markermoleküle sind im Stand der Technik bekannt und können nach bekannten Verfahren hergestellt oder im Handel bezogen werden. Einige Beispiele für kommerziell erhältliche Substanzen sind: Dansylcadaverin, Fluoresceincadaverin, 5-Aminoeosin, Biotinylcadaverin, Tetramethylrhodamincadaverin sowie "Oregon Green"- und "Alexa Fluor"- Cadaverin (Hersteller: Molecular Probes, siehe z. B. den Katalog dieses Herstellers für weitere Beispiele).

Die Proteinmodifizierung kann jedoch auch die Kopplung des Proteins mit einer nicht-markierten reaktiven oder stabilisierenden Gruppierung beinhalten. Einige, nicht beschränkende, Beispiele für solche Gruppierungen sind PEG (Polyethylenglycol) und Derivate davon, Dextran und Derivate davon sowie Stärke und Stärkederivate. Da es bei der Modifizierung, insbesondere Stabilisierung, von Proteinen mit solchen Gruppierungen oft auf die genaue Position der Modifikation im Protein ankommt, kann hier die Reaktionsführung unter selektiver gesteuerter Transglutaminasekatalyse spezielle Vorteile gegenüber der üblichen chemischen Modifizierung bieten.

Falls das zu modifizierende Protein keine reaktionsfähigen Lysin- oder Glutaminreste für die Transglutaminase-katalysierte Modifizierungsreaktion enthält, so können durch im Stand der Technik bekannte Verfahren geeignete Reste eingeführt werden. Beispielsweise kann durch molekulargenetische Methoden gezielt ein geringfügig verändertes Protein hergestellt werden, das eine Bindungsstelle für Transglutaminasen enthält. Alternativ kann das Zielprotein auch mit einer Peptidsequenz, die eine solche Bindungsstelle enthält, fusioniert werden, um dadurch zu einem Substrat für Transglutaminase zu werden.

### FIGURENBESCHREIBUNG:

- **Fig. 1A**: Transglutaminase-katalysierte Vernetzung von Proteinen
- **Fig. 1B**: Transgutaminase-katalysierte Kopplung von Proteinen mit Markermolekülen
- **Fig. 1C**: Markierung von Lysinresten eines Proteins mit nachweisbaren Peptidderivaten
- **Fig. 1D**: kovalente Bindung eines Transglutaminase-Inhibitors an das Cystein des aktiven Zentrums der Transglutaminase
- **Fig. 1 E**: Schema der Herstellung eines Transglutaminase-Inhibitors der Formel I
- **Fig. 2**: Fluoreszenzaufnahme der elektrophoretisch aufgetrennten Produkte nach Transglutaminase-katalysierter Markierung eines Proteins und Beendigung der Markierungsreaktion durch einen Inhibitor unter den Bedingungen von Beispiel 1

| Spurbelegung: | |
|---|---|
| Spur 1 | Prestained Proteinmolekulargewichtsmarker |
| Spur 2 | unmarkiertes Protein |
| Spur 3 | 2 Min., direkt nach Inhibitorzugabe |
| Spur 4 | 2 Min., nach Inhibitorabtrennung + 60 Min. RT |
| Spur 5 | 10 Min., direkt nach Inhibitorzugabe |
| Spur 6 | 10 Min., nach Inhibitorabtrennung + 60 Min. RT |
| Spur 7 | 20 Min., direkt nach Inhibitorzugabe |
| Spur 8 | 20 Min., nach Inhibitorabtrennung + 60 Min. RT |
| Spur 9 | 15 Min., vor PD-10-Säule |
| Spur 10 | 15 Min., nach PD-10-Säule |

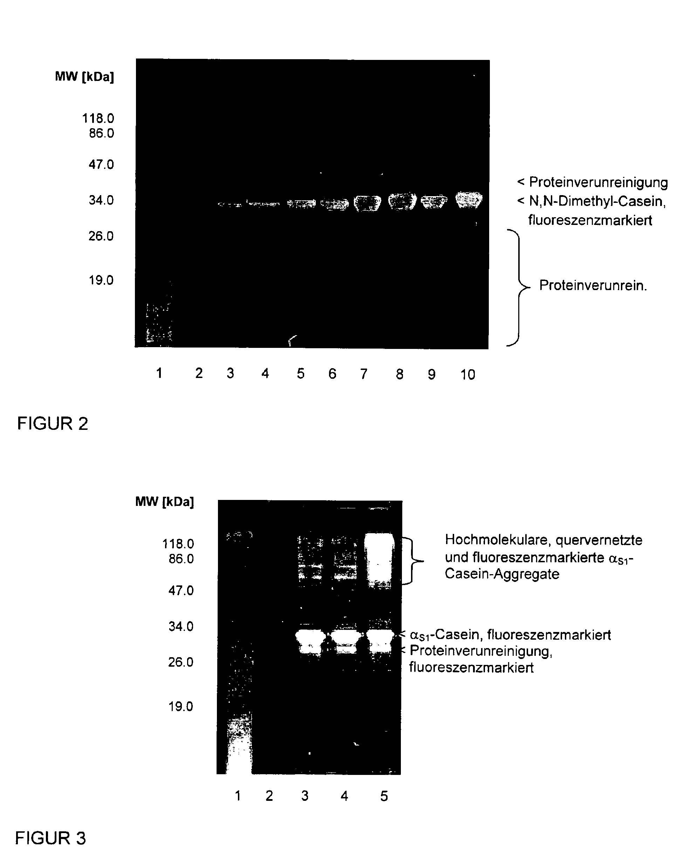

- **Fig. 3**: Fluoreszenzaufnahme der elektrophoretisch aufgetrennten Produkte nach Transglutaminase-katalysierter Markierung eines Proteins und Beendigung der Markierungsreaktion durch einen Inhibitor unter den Bedingungen von Beispiel 2

| Spurbelegung: | |
|---|---|
| Spur 1 | Prestained Proteinmolekulargewichtsmarker |
| Spur 2 | unmarkiertes Protein |
| Spur 3 | 10 Min., direkt nach Inhibitorzugabe |
| Spur 4 | 10 Min., nach Inhibitorzugabe + 30 Min. RT |
| Spur 5 | 10 Min., nach PD-10-Säule |

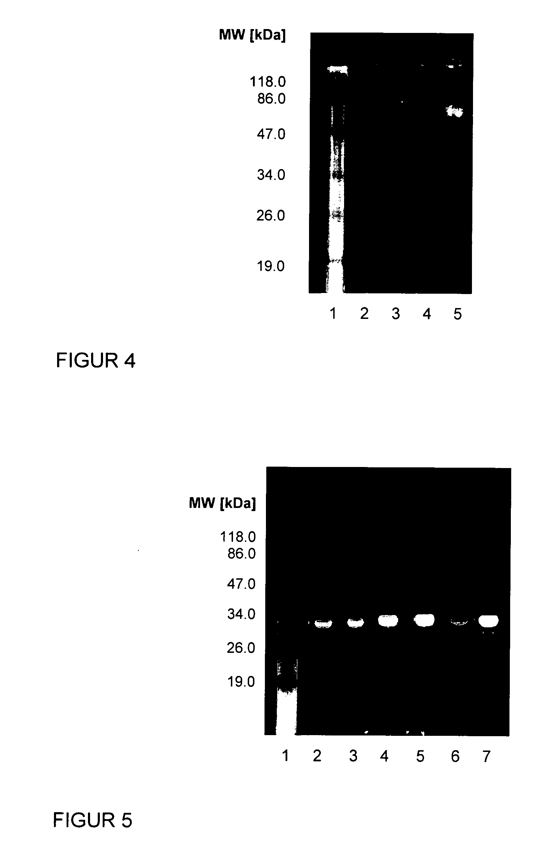

- **Fig. 4**: Fluoreszenzaufnahme der elektrophoretisch aufgetrennten Produkte nach Transglutaminase-katalysierter Markierung eines Proteins und Beendigung der Markierungsreaktion durch einen Inhibitor unter den Bedingungen von Beispiel 3

| Spurbelegung: | |
|---|---|
| Spur 1 | Prestained Proteinmolekulargewichtsmarker |
| Spur 2 | 5 Min., direkt nach Inhibitorzugabe |
| Spur 3 | 5 Min., nach Inhibitorzugabe + 30 Min. RT nach Abtrennung des Inhibitors |
| Spur 4 | 5 Min., vor PD-10-Säule |
| Spur 5 | 5 Min., nach PD-10-Säule |

- **Fig. 5**: Fluoreszenzaufnahme der elektrophoretisch aufgetrennten Produkte nach Transglutaminase-katalysierter Markierung eines Proteins und Beendigung der Markierungsreaktion durch einen Inhibitor unter den Bedingungen von Beispiel 4

| Spurbelegung: | |
|---|---|
| Spur 1 | Prestained Proteinmolekulargewichtsmarker |
| Spur 2 | 2 Min., direkt nach Inhibitorzugabe |
| Spur 3 | 2 Min., nach Inhibitorzugabe + 30 Min. RT |
| Spur 4 | 5 Min., direkt nach Inhibitorzugabe |
| Spur 5 | 5 Min., nach Inhibitorzugabe + 30 Min. RT |
| Spur 6 | 1 Min., vor PD-10-Säule |
| Spur 7 | 1 Min., nach PD-10-Säule |

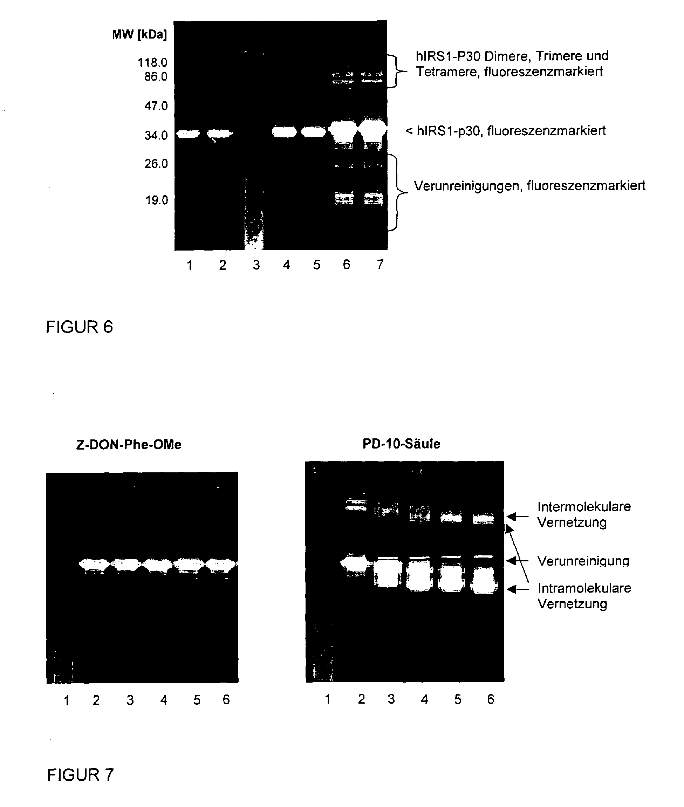

- **Fig. 6**: Fluoreszenzaufnahme der elektrophoretisch aufgetrennten Produkte nach Transglutaminase-katalysierter Markierung eines Proteins und Beendigung der Markierungsreaktion durch einen Inhibitor unter den Bedingungen von Beispiel 5

| Spurbelegung: | |
|---|---|
| Spur 1 | 2 Min., direkt nach Inhibitorzugabe |
| Spur 2 | 2 Min., nach Inhibitorzugabe + 30 Min. RT |
| Spur 3 | Prestained Proteinmolekulargewichtsmarker |
| Spur 4 | 5 Min., direkt nach Inhibitorzugabe |
| Spur 5 | 5 Min., nach Inhibitorzugabe + 30 Min. RT |
| Spur 6 | 10 Min., direkt nach Inhibitorzugabe |
| Spur 7 | 10 Min., nach Inhibitorzugabe + 30 Min. RT |

- **Fig. 7**: Fluoreszenzaufnahme der elektrophoretisch aufgetrennten Produkte nach Transglutaminase-katalysierter Markierung eines Proteins und Beendigung der Markierungsreaktion durch einen Inhibitor unter den Bedingungen von Beispiel 6

| Spurbelegung: | |
|---|---|
| Spur 1 | Prestained Proteinmolekulargewichtsmarker |
| Spur 2 | 5 Min., direkt nach Inhibitorzugabe |
| | bzw. direkt nach PD-10 Säule |
| Spur 3 | nach 30 Min. RT |
| Spur 4 | nach 1 h RT |
| Spur 5 | nach 2 h RT |
| Spur 6 | nach 4 h RT |

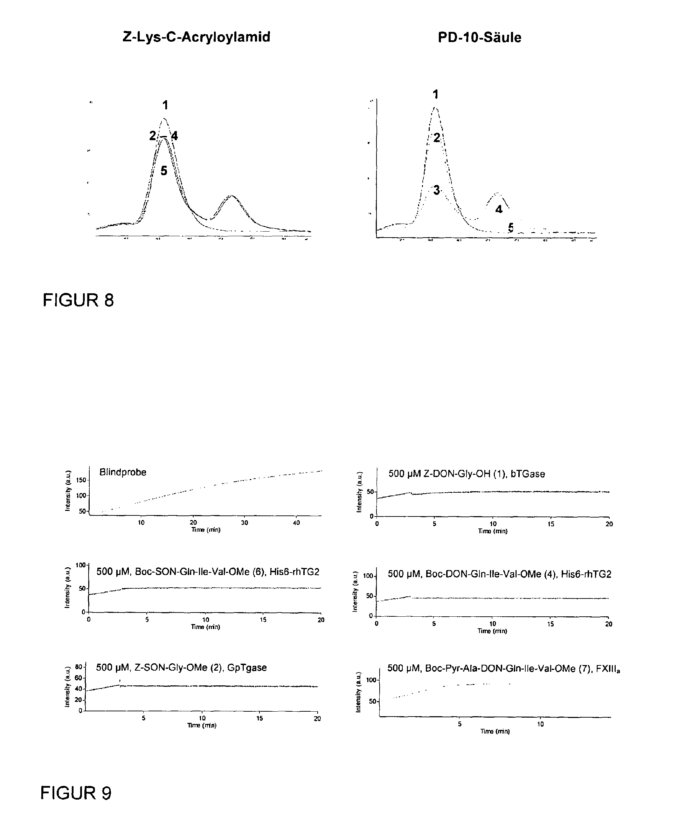

- **Fig. 8**: RP-HPLC-Chromatogramme der aufgetrennten Produkte nach Transglutaminasekatalysierter Markierung eines Proteins und Beendigung der Markierungsreaktion durch einen Inhibitor bzw. Abtrennung der TG durch eine PD-10-Säule unter den Bedingungen von Beispiel 7

| | |
|---|---|
| Nr.1 | hLeptin, 25 µg |
| Nr.2 | 5 Min., direkt nach Inhibitorzugabe bzw. direkt nach PD-10 Säule |
| Nr.3 | 5 Min. + 30 Min. RT |
| Nr.4 | 5 Min. + 60 Min. RT |
| Nr.5 | 5 Min. + 10 Tage -20°C und eine Stunde RT |

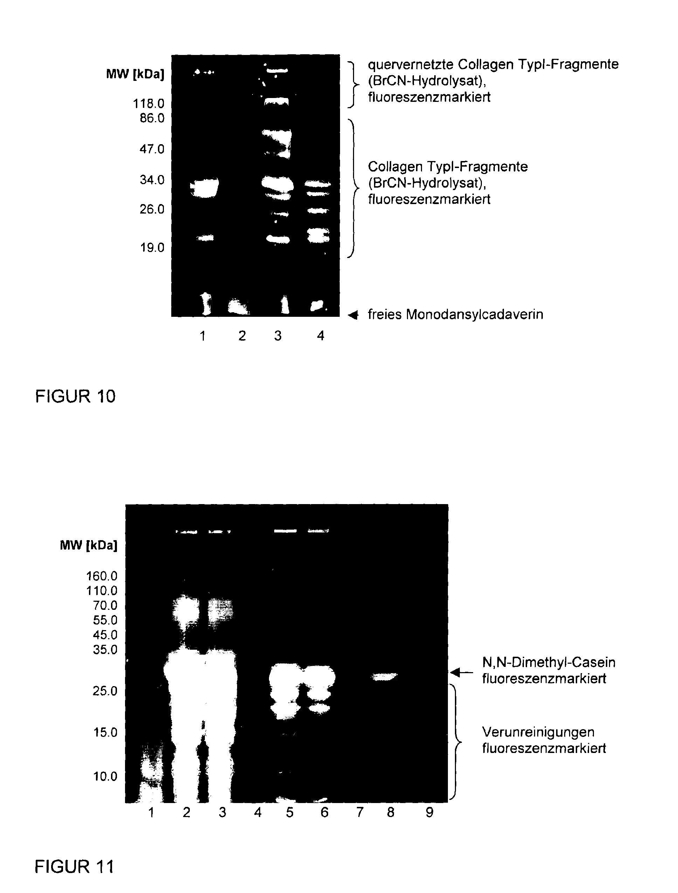

- **Fig. 9**: Fluoreszenzspektrogramme der Produkte nach Transglutaminase-katalysierter Fluoreszenzmarkierung eines Proteins und Beendigung der Markierungsreaktion durch einen Inhibitor unter den Bedingungen von Beispiel 8
- **Fig. 10**: Fluoreszenzaufnahme der elektrophoretisch aufgetrennten Produkte nach Markierung eines Proteins mit verschiedenen Transglutaminasen und Beendigung der Markierungsreaktionen durch einen Inhibitor unter den Bedingungen von Beispiel 9

| Spurbelegung: | |
|---|---|
| Spur 1 | 4 h, mit His₆-rhTG2 markiert |
| Spur 2 | Prestained Proteinmolekulargewichtsmarker |
| Spur 3 | 4 h, mit hFaktor Xllla markiert |
| Spur 4 | 4 h, mit bTGase markiert |

- **Fig. 11**: Fluoreszenzaufnahme der elektrophoretisch aufgetrennten Produkte nach Markierung eines Proteins mit verschiedenen Transglutaminasen und Beendigung der Markierungsreaktionen durch einen Inhibitor unter den Bedingungen von Beispiel 10

| Spurbelegung: | |
|---|---|
| Spur 1 | Prestained Proteinmolekulargewichtsmarker |
| Spur 2 | bTGase, 60 Min. |
| Spur 3 | bTGase, 30 Min. |
| Spur 4 | - |
| Spur 5 | gpTGase, 60 Min. |
| Spur 6 | gpTGase, 30 Min. |
| Spur 7 | - |
| Spur 8 | hFXIIIa, 60 Min. |
| Spur 9 | hFXIIIa, 30 Min. |

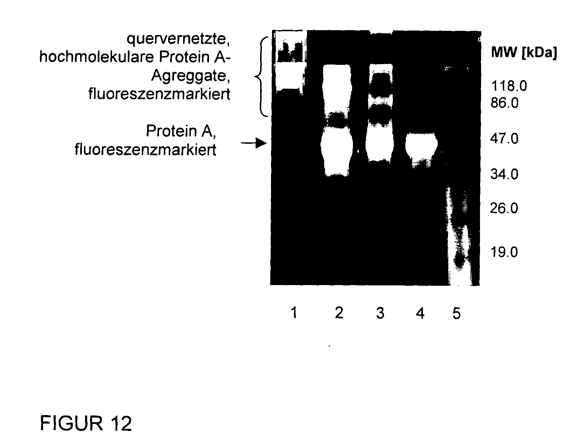

- **Fig. 12**: Fluoreszenzaufnahme der elektrophoretisch aufgetrennten Produkte nach Markierung eines Proteins mit verschiedenen Transglutaminasen und Beendigung der Markierungsreaktionen durch einen Inhibitor unter den Bedingungen von Beispiel 11

| Spurbelegung: | |
|---|---|
| Spur 1 | bTGase-Labeling, 4 h |
| Spur 2 | gpTGase-Labeling, 4 h |
| Spur 3 | His₆-rhTG2 -Labeling, 4 h |
| Spur 4 | hfaktor XIIa-Labeling, 4 h |
| Spur 5 | Prestained Proteinmolekulargewichtsmarker |

### BEISPIELE FÜR DIE SYNTHESE VON INHIBITOREN:

### SYNTHESEBEISPIEL 1

### N^{α}-Benzyloxycarbonyl-L-(6-diazo-5-oxonorleucinyl)glycinmethylester

400 mg N^{α}-Benzyloxycarbonyl-L-glutamyl-glycinmethylester (1,135 mmol), nach Standardmethoden der Peptidchemie hergestellt, werden in 15 ml THF_{abs} gelöst und auf -20 °C gekühlt. Unter Argonatmosphäre werden 778 µl Isobutylchlorformiat zugesetzt (5,67 mmol). Nach 5 Min. wird dieser Lösung eine frisch hergestellte Lösung aus Diazomethan in Diethylether zugesetzt (aus 5,6 g Diazald und 36 ml Diethylether) zugesetzt. Innerhalb von 12 Stunden wird allmählich auf Raumtemperatur aufgetaut und danach für weitere 8 Stunden bei Raumtemperatur gerührt. Überschüssiges Diazomethan wird im Stickstoffstrom entfernt und das Lösungsmittel im Vakuum entfernt. Die Reinigung erfolgt durch Chromatographie an Kieselgel (Säule: 2,3 x 28 cm, Laufmittel: Dichlormethan/Methanol = 98/2, R_{f} = 0,25).
Ausbeute: 363 mg (85 % d. Th.)
500-MHz-¹H-NMR-cosy (DMSO_{d6}): δ[ppm] = 8,34 (t, 1H), 7,45 (d, 1H), 7,31-7,38 (m, 5H), 6,00 (br.s., 1H), 5,03 (dd, 2H), 4,03 (m, 1 H), 3,89 (dd, 1 H), 3,79 (dd, 1 H), 3,63 (s, 3H), 2,39 (m, 2H), 1,90-1,94 (m, 1 H), 1,75-1,79 (m, 1 H)
ESI-MS: 399,0 [M+Na]⁺, 371,1 [M-N₂+Na]⁺

### SYNTHESEBEISPIEL 2

### N^{α}-Benzyloxycarbonyl-L-(6-diazo-5-oxonorleucinyl)glycin (1)

50 mg N^{α}-Benzyloxycarbonyl-L-(6-diazo-5-oxonorleucinyl)glycinmethylester (0,133 mmol) werden in 5 ml Methanol gelöst und bei 0 °C mit 200 µl wässriger NaOH (1 M)-Lösung versetzt (0,2 mmol). Nach einer Stunde wird das Eisbad entfernt und über Nacht bei Raumtemperatur weiter gerührt. Die Lösung wird mit wässriger KHSO₄-Lösung (0,5 M) neutralisiert und das Lösungsmittel im Vakuum entfernt. Die Reinigung erfolgt durch Chromatographie an Kieselgel (Säule: 1,7 x 16 cm, Laufmittel: Dichlormethan/Methanol Stufengradient 80/20 auf 1/1, R_{f} = 0,25).
Ausbeute: 24 mg (49,8 % d. Th.)
500-MHz-¹H-NMR-cosy (DMSO_{d6}): δ[ppm] = 7,66-7,67 (m, 2H), 7,38-7,51 (m, 5H), 6,18 (br.s., 1 H), 5,15 (dd, 2H), 4,10 (m, 1 H), 2,49 (m, 2 H), 2,00-2,13 (m, 1 H), 1,80-1,93 (m, 1 H)
ESI-MS: 385,1 [M+Na]⁺, 357,2 [M-N₂+Na]⁺

### SYNTHESEBEISPIEL 3

### N^{α}-Benzyloxycarbonyl-L-(6-dimethylsulfonium-5-oxonorleucinyl)glycinmethylester (Bromidsalz) (2)

50 mg N^{α}-Benzyloxycarbonyl-L-(6-diazo-5-oxonorleucinyl)glycinmethylester (0,133 mmol) werden in 10 ml THF_{abs} gelöst. Nach Zusatz katalytischer Mengen Bromthymolblau wird unter Argonatmosphäre bei 0 °C 3,3 % HBr in Essigsäure zugetropft, bis der Indikator nach violett umschlägt. Sofort werden 50 ml Wasser zugesetzt und das Produkt zweimal mit je 25 ml Ethylacetat extrahiert. Die organische Phase wird mit Wasser gewaschen, bis die Farblosigkeit der Waschlösung die vollständige Entfernung des Indikators anzeigt und danach mit Na₂SO₄ getrocknet. Nach Entfernen des Lösungsmittels im Vakuum und Trocknen im Hochvakuum erhält man 58 mg N^{α}-Benzyloxycarbonyl-L-(6-brom-5-oxonorleucinyl)glycinmethylester als leicht gelben Feststoff. Zur weiteren Umsetzung zur Titelverbindung wird die Substanz in 2 ml Aceton gelöst und mit 500 µl Dimethylsulfid (9,5 mmol) versetzt. Nach Rühren für 48 Stunden wird das Lösungsmittel im Vakuum entfernt und die erhaltene braune Substanz durch Chromatographie an Kieselgel gereinigt. (Säule: 3,2 x 3 cm, Laufmittel: Dichlormethan/Methanol 80/20, R_{f} = 0,08)
Ausbeute: 14 mg (21%)
ESI-MS: 411,1 [M]⁺

### SYNTHESEBEISPIEL 4

### N^{α}-Benzyloxycarbonyl-L-(6-diazo-5-oxonorleucinyl)-L-phenylalaninmethylester (3)

Eine Lösung aus 400 mg N^{α}-Benzyloxycarbonyl-L-glutamyl-phenylalaninmethylester, nach Standardmethoden der Peptidchemie hergestellt, (0,904 mmol) in 20 ml THF wird auf -20 °C gekühlt und unter Argonatmosphäre mit 770 µl Ethyldiisopropylamin (4,5 mmol) und anschließend mit 619 µl Isobutylchlorformiat versetzt (4,5 mmol). Nach fünf Minuten wird eine frisch hergestellte Diazomethanlösung (4 g Diazald / 20 ml Diethylether) zugesetzt und bei 0°C für eine Stunde und nachfolgend für 12 Stunden bei Raumtemperatur gerührt. Das Lösungsmittel wird im Vakuum entfernt und das Rohprodukt durch Chromatographie an Kieselgel gereinigt ((Säule: 2,3 x 40 cm, Laufmittel: Dichlormethan/Methanol 98/2, R_{f} = 0,16).
Ausbeute: 298 mg (68%)
ESI-MS: 489,3 [M+Na]⁺, 461,3 [M-N₂+Na]⁺

### SYNTHESEBEISPIEL 5

### Nα-Benzyloxycarbonyl-L-(6-dimethylsulfonium-5-oxonorleucinyl)phenylalaninmethylester (Bromid-Salz) (4)

100 mg N^{α}-Benzyloxycarbonyl-L-(6-diazo-5-oxonorleucinyl)phenylalaninmethylester (0,207 mmol) werden in 5 ml THF_{abs} gelöst. Nach Zusatz katalytischer Mengen Bromthymolblau wird unter Argonatmosphäre bei 0 °C 3,3 % HBr in Essigsäure zugetropft, bis der Indikator nach violett umschlägt. Sofort werden 30 ml Wasser zugesetzt und das Produkt zweimal mit je 20 ml Ethylacetat extrahiert. Die organische Phase wird mit Wasser gewaschen, bis die Farblosigkeit der Waschlösung die vollständige Entfernung des Indikators anzeigt, und danach mit Na₂SO₄ getrocknet. Nach Entfernen des Lösungsmittels im Vakuum wird ohne weitere Reinigung die Umsetzung zur Titelverbindung durch Lösen der Substanz in 2 ml Aceton und Zusatz von 760 µl Dimethylsulfid (10,3 mmol) durchgeführt. Nach Rühren für 48 Stunden wird das Lösungsmittel im Vakuum entfernt und die erhaltene braune Substanz durch Chromatographie an Kieselgel gereinigt. (Säule: 3,2 x 2,5 cm, Laufmittel: Methanol, R_{f} = 0,04)
Ausbeute: 54 mg (45%)
ESI-MS: 501,1 [M]⁺

### SYNTHESEBEISPIEL 6

### N^{α}-tert.-Butyloxycarbonyl-L-(6-diazo-5-oxonorleucinyl)-L-glutaminyl-L-isoleucinyl-L-valinmethylester (5)

884 mg N^{α}-tert.-Butyloxycarbonyl-L-glutamyl-L-glutaminyl-L-isoleucinyl-L-valinmethylester, nach Standardmethoden der Peptidchemie hergestellt, (1,47 mmol) werden in 6 ml DMSO gelöst und mit 54 ml THF_{abs} verdünnt. Bei -20 °C werden unter Argonatmosphäre nacheinander 1,25 ml Diisopropylethylamin (7,35 mmol) und 1,01 ml Isobutylchlorformiat (95%ig) zugesetzt. Nach fünf Minuten Rühren bei -20 °C versetzt man die aktivierte Carbonsäure mit 32 mmol frisch hergestellter Diazomethanlösung in 50 ml Diethylether und rührt bei 0 °C für eine Stunde und nachfolgend für 12 Stunden bei Raumtemperatur unter Argonathmosphäre. Das Lösungsmittel wird im Vakuum entfernt und das Rohprodukt durch Chromatographie an Kieselgel gereinigt (Säule: 3,0 x 39,5 cm, Laufmittel: Dichlormethan/Methanol 93,5/6,5, R_{f} = 0,13).
Ausbeute: 480 mg (52 %)
ESI-MS: 648,4 {M+Na]⁺, 620,4 [M-N₂+Na]⁺, 606,4 [M(CH₂N₂)+Na]⁺, 624,3 [M-H]⁻, 582,3 [M-(CH₂N₂)]⁻, 550,3 [M-t-BuOH]⁻

500-MHz-¹H-NMR-cosy (DMSO_{d6}): δ[ppm] = 8,15 (d, 1H), 7,93 (d, 1H), 7,82 (d, 1H), 7,20 (s, 1 H), 6,95 (d, 1 H), 6,95 (d, 1 H), 6,75 (s, 1 H), 6,03 (br.s., 1 H), 4,33-4,25 (m, 2H), 4,13 (dd, 1H), 3,61 (s, 3H), 3,90 (m, 1H), 2,38-2,28 (m, 2H), 2,13-2,0 (m, 3H), 1,90-1,79 (m, 2H), 1,76-1,65 (m, 3H), 1,45-1,37 (m, 1H), 1,38 (s, 9H), 0,91-0,77 (m, 12H).

### SYNTHESEBEISPIEL 7

### N^{α}-tert.-Butyloxycarbonyl-L-(6-dimethylsulfonium-5-oxonorleucinyl)-L-glutaminyl-L-isoleucinyl-L-valinmethylester (6) (Bromidsalz)

100 mg N^{α}-tert.-Butyloxycarbonyl-L-(6-diazo-5-oxonorleucinyl)-L-glutaminyl-L-isoleucinyl-L-valinmethylester (0,16 mmol) werden in 50 ml THF_{abs} gelöst und nach Zusatz einer katalytischen Menge Bromthymolblau auf -10 °C gekühlt. Tropfenweise wird eine Lösung von 3 % HBr in Eisessig zugesetzt, bis der Indikator nach violett wechselt. Durch Zusatz von 20 ml 3 %iger NaHCO₃-Lösung wird die Reaktion abgestoppt und die Lösung sofort mit 100 ml Ethylacetat versetzt. Die organische Phase wird mit Wasser gewaschen, bis die Farblosigkeit der Waschlösung die vollständige Entfernung des Indikators anzeigt und danach mit Na₂SO₄ getrocknet. Nach Entfernen des Lösungsmittels im Vakuum erfolgt die Umsetzung zur Titelverbindung ohne weitere Reinigung. Dazu wird die erhaltene Substanz in 30 ml Aceton gelöst und nachfolgend 500 µl Dimethylsulfid zugesetzt. Nach Rühren für 48 Stunden wird das Lösungsmittel im Vakuum entfernt und die erhaltene braune Substanz durch Chromatographie an Kieselgel gereinigt. (Säule: 5 x 3 cm, Laufmittel: Methanol, R_{f}=0,04)
Ausbeute: 44 mg (37% d. Th.)
ESI-MS: 682,4 [M-H+Na]⁺, 660,4 [M]

### SYNTHESEBEISPIEL 8

### N^{α}-tert.-Benzyloxycarbonyl-L-pyroglutaminyl-L-alaninyl-L-(6-diazo-5-oxonorleucinyl)-L-glutaminyl-L-isoleucinyl-L-valinmethylester (7)

50 mg N^{α}-tert.-Benzyloxycarbonyl-L-pyroglutaminyl-L-alaninyl-L-glutamyl-L-glutaminyl-L-isoleucinyl-L-valinmethylester (0,061 mmol), nach Standardverfahren der Peptidchemie hergestellt, werden in 700 µl DMSO gelöst und mit 2,5 ml THF_{abs} verdünnt. Bei -20 °C werden unter Argonatmosphäre nacheinander 52 µl Diisopropylethylamin (0,306 mmol) und 41,9 µl Isobutylchloroformiat (0,306 mmol) zugegeben. Nach 5 Min. Rühren bei -20 °C werden 7 ml frisch hergestellte Diazomethanlösung (~1,7 mmol) zugesetzt und bei 0°C weitergerührt. Über Nacht lässt man die Lösung allmählich auf Raumtemperatur kommen. Nach Entfernung des Lösungsmittels im Vakuum wird der erhaltene Feststoff durch Chromatographie an Kieselgel gereinigt. (Säule: 2,3 x 27,5 cm, Laufmittel: Dichlormethan/Methanol 85/15, R_{f} = 0,51).
Ausbeute: 20mg (39%)
ESI-MS: 864,4 [M+Na]⁺, 836,5 [M-N₂+Na]⁺

### Beispiele zur Reaktionskontrolle: Einstellung eines definierten Markierungsgrades in Transglutaminase-katalysierten Markierungsreaktionen über selektive irreversible Inhibitoren

### Beschreibung der Versuchsanordnung

Alle nachfolgend beschriebenen Experimente wurden nach dem gleichen Standardprotokoll durchgeführt. Dabei werden das jeweilige Zielprotein und das farbstofftragende amin- oder glutaminhaltige Substrat in einem geeigneten Puffer vorgelegt und für 5 Min. auf 37°C temperiert. Anschließend wird durch Zugabe von Transglutaminase die Reaktion gestartet. Nach variierenden Inkubationszeiten, durch die jeweils verschiedene Markierungsgrade erreicht werden, wird durch Zugabe eines Inhibitors die Transglutaminase vollständig und irreversibel inaktiviert (Reaktionsabbruch 1). In Kontrollexperimenten wird die Markierungsreaktion durch Abtrennung des ungebundenen, farbstofftragenden Substrates unterbrochen (Reaktionsabbruch 2). Die Abtrennung des ungebundenen Farbstoffes erfolgt unter Normaldruck über eine PD-10-Säule (Amersham Biosciences). Dieses allgemein einsetzbare Verfahren zur schnellen Abtrennung kleiner Moleküle von Makromolekülen wie Proteinen basiert auf dem Prinzip der Gelpermeationschromatographie und kann als das vorteilhafteste Verfahren zur Reaktionskontrolle nach dem Stand der Technik angesehen werden. Andere Verfahren, mit denen die weitere katalytische Markierungsreaktion abgebrochen werden könnte, sind weniger geeignet oder nicht allgemein einsetzbar (z. B. thermische, unspezifische chemische oder affinitätschromatographische Methoden). Die Gelpermeationschromatographie wurde jeweils so schnell wie technisch machbar durchgeführt. Dazu wird auf die vorkonditionierte PD-10-Säule 2,5 ml des Reaktionsvolumens aufgetragen und die Lösung vollständig in das Trennmaterial eingebracht. Anschließend wird das Protein mit 3,5 ml Puffer eluiert. Die niedermolekularen Substanzen verbleiben auf der Säule. Der Gesamtprozess benötigt eine Zeitspanne von mindestens 3 Minuten. Während dieser Zeit kann Transglutaminase weiter auf das Zielprotein einwirken. Außerdem bleibt die eingesetzte Transglutaminase in aktiver Form in der erhaltenen Proteinlösung zurück und kann Proteine, die sowohl als Glutamin als auch als Lysindonor fungieren, sowohl intra- als auch intermolekular vernetzen und dadurch höhere bzw. nicht funktionelle Proteinspezies zusammen mit dem Zielprotein im Produkt generieren.

In allen Beispielen, in denen die katalytische Reaktion durch Zugabe der Inhibitoren abgebrochen wird, wurde dieser Inhibitor nach der jeweils angegebenen Zeit, die der Reaktionsansatz bei Raumtemperatur stehen gelassen wurde, ebenfalls durch Gelpermeationschromatographie abgetrennt. Die Transglutaminasen erhalten hierbei ihre Aktivität nicht zurück, wodurch der streng irreversible Charakter der Inhibition experimentell belegt wird.

### Verwendete Begriffe und Abkürzunqen:

| **Allgemeine Begriffe:** | |
|---|---|
| RT | Raumtemperatur |
| SDS-PAGE | Natriumdodecylsulfat-Polyacrylamid-Gelelektrophorese |
| DOL | Markierungsgrad ("degree of labeling") |

| **Verwendete Transglutaminasen:** | |
|---|---|
| His₆rhTG2 | rekombinante humane Transglutaminase 2, His₆-tag-N-terminal |
| bTGase | bakterielle Transglutaminase aus *Streptomyces mobaraensis E. C. 2.3.3.13* |
| hFaktor Xllla | humaner Blutgerinnungsfaktor XIII (Thrombin-aktiviert) |
| GpTGase | Meerschweinchenlebertransglutaminase |

| **Verwendete Inhibitoren:** | |
|---|---|
| N^{α}-Benzyloxycarbonyl-L-(6-diazo-5-oxonorleucinyl)glycin (1) | Z-DON-Gly-OH |
| N^{α}-Benzyloxycarbonyl-L-(6-dimethylsulfonium-5-oxonorleucinyl)glycinmethylester (Bromidsalz) (2) | Z-SON-Gly-OMe |
| N^{α}-Benzyloxycarbonyl-L-(6-diazo-5-oxonorleucinyl)-L-phenylalaninmethylester (3) | Z-DON-Phe-OMe |
| N^{α}-Benzyloxycarbonyl-L-(6-dimethylsulfonium-5-oxonorleucinyl)-phenylalaninmethylester (Bromidsalz) (4) | Z-SON-Phe-OMe |
| N"-tert.-Butyloxycarbonyl-L-(6-diazo-5-oxonorleucinyl)-L-glutaminyl-L-isoleucinyl-L-valinmethylester (5) | Boc-DON-Gln-Ile-Val-OMe |
| N"-tert.-Butyloxycarbonyl-L-(6-dimethylsulfonium-5-oxonorleucinyl)-L-glutaminyl-L-isoleucinyl-L-valinmethylester (6) | Boc-SON-Gln-Ile-Val-OMe |
| N^{α}-tert.-Benzyloxycarbonyl-L-pyroglutaMin.y-L-Alaninyl-L-(6-diazo-5-oxonorleucinyl)-L-glutaMin.yl-L-isoleucinyl-L-valinmethylester (7) | Boc-Pyr-Ala-DON-Gln-Ile-Val-OMe |
| N^{α}-Benzyloxycarbonyl-L-serinyl(O-chloracetyl)glycin | Z-Ser(OAcCl)-Gly-OH |
| N'-Benzyloxycarbonyl-L-lysinyl(e-acryloyl)glycin | Z-Lys(Acryloyl)-Gly-OH |
| N-(N^{α}-Benzyloxycarbonyl-L-phenylalaninyl)-(4-amino-2-oxo-dimethylsulfonium)pentan, Bromidsalz | Z-Phe-NH-(CH₂)₃-CO-S⁺(CH₃)₂Br⁻ |

| **Verwendete Substrate**: | |
|---|---|
| N-(N^{α}-Benzyloxycarbonyl-L-glutaminyl-glycyl)-5-N-(5'-N', N'-dimethylamino-1'-naphthalinsulfonyl)diamidopentan | Z-Gln-Gly-CAD-DNS |
| N-(N^{α}-Benzyloxycarbonyl -L-glutaminyl-glycyl)-5-N-(N,N, N', N'-Tetramethylrhodaminyl)-diamidopentan | Z-Gln-Gly-DAH-TAMRA |
| N-5-Aminopentyl-5-dimethylamin-1-naphthalinsulfonamid Monodansylcadaverin | |

In den folgenden 5 Beispielen werden Transglutaminase-katalysierte Markierungsreaktionen mit verschiedenen Proteinen und verschiedenen Inhibitoren durchgeführt und gezeigt, dass diese Reaktionen mit den erfindungsgemäßen Inhibitoren steuerbar sind.

### BEISPIEL 1

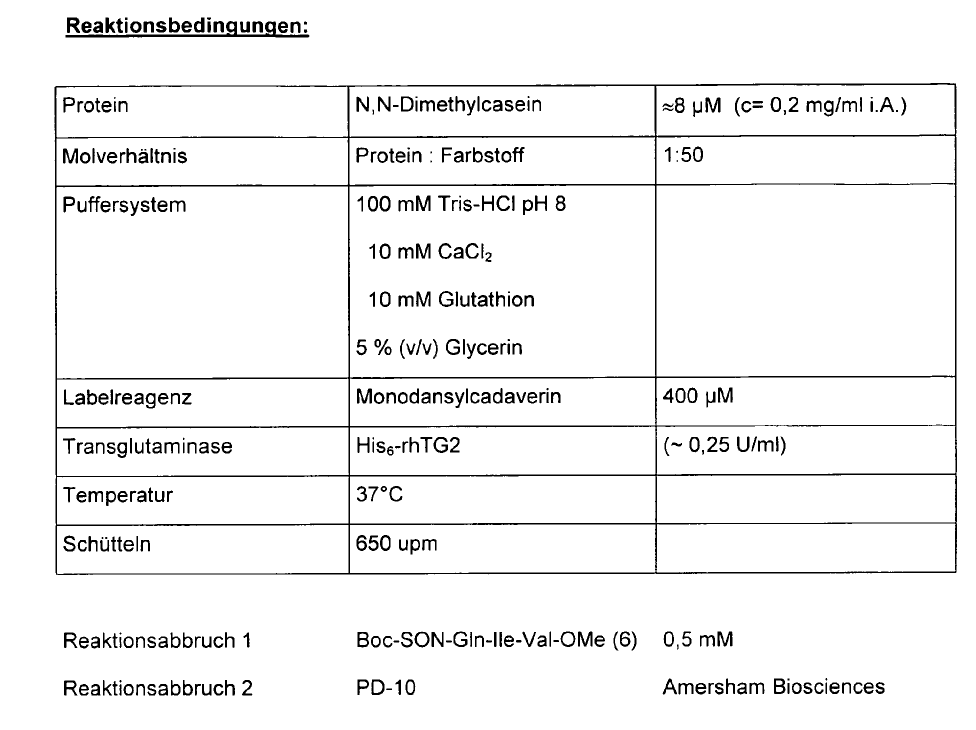

### Versuchsbeschreibung:

In drei von vier identischen Ansätzen werden nach 2, 10 und 20 Minuten Reaktionszeit die Reaktion durch Zugabe des Inhibitors Boc-SON-Gln-Ile-Val-OH abgestoppt (Reaktionsabbruch Nr.1). Unmittelbar nach dem Abbruch der katalytischen Reaktion wird eine Probe entnommen und durch SDS-PAGE charakterisiert. Eine weitere Probe wird nach Zugabe des Inhibitors zusätzlich 20 Minuten bei RT stehen gelassen, bevor die Abtrennung von nicht kovalent gebundenem Inhibitor und freiem Farbstoff über eine Gelpermeationschromatographie erfolgt, und anschließend für 60 Min. bei RT aufbewahrt. Aus dieser Lösung wird eine zweite SDS-PAGE-Probe entnommen.

Die Abtrennung des ungebundenen Farbstoffes durch Gelpermeationschromatographie wird als Alternativverfahren nach 15 Minuten Reaktionszeit durchgeführt. Auch hier werden direkt vor und nach der Abtrennung des freien Farbstoffes SDS-PAGE-Proben zur Überprüfung entnommen.

### Analytik:

| | |
|---|---|
| Polyacrylamidgel | Trenngel 12,5 %ig, Sammelgel 5,0 %ig |
| Puffersystem | Tris-Glycin |
| Auftragsmenge | ~ 1,5 µg / Spur |
| Elektrophorese-System | BioRad Mini-PROTEAN® 3 cell |
| Visualisierung | Videodokumentationsanlage LTF, Fluoreszenzaufnahme UV-Anregung bei 280-350 nm |

Die Fluoreszenzaufnahme des Gels ist in Fig. 2 gezeigt.

### Auswertung:

Im vorliegenden Beispiel wurde ein Protein verwendet, welches durch chemische Veränderung keine freien Lysinreste an der Oberfläche trägt. Es kann also durch Transglutaminase keine intra- oder intermolekulare Vernetzung eintreten. In diesem stark vereinfachten Fall kann somit die reine Markierungsreaktion bzw. der zunehmende Markierungsgrad des Zielproteins gut erkannt werden. Mit längerer Reaktionszeit nimmt die Menge an mehrfach markiertem N,N-Dimethylcasein (Zielprotein) deutlich zu. Im Verlaufe des Prozesses der Abtrennung des ungebundenen Farbstoffes durch Gelpermeationschromatographie ist eine erkennbare Zunahme an gebundenem Fluoreszenzlabel festzustellen. Ein definierter Markierungsgrad (DOL) ist durch dieses Verfahren nicht realisierbar. Im Gegensatz dazu kann durch das sehr schnelle Abstoppen der Reaktion durch einen erfindungsgemäßen Inhibitor ein gewünschter DOL sehr genau eingestellt werden. Eine weitere katalytische Reaktion findet nach Zugabe des Inhibitors nicht statt. In Gegenwart eines großen Überschusses an ungebundenem Farbstoff ist keine Zunahme von gebundenem Farbstoff erkennbar.

### BEISPIEL 2

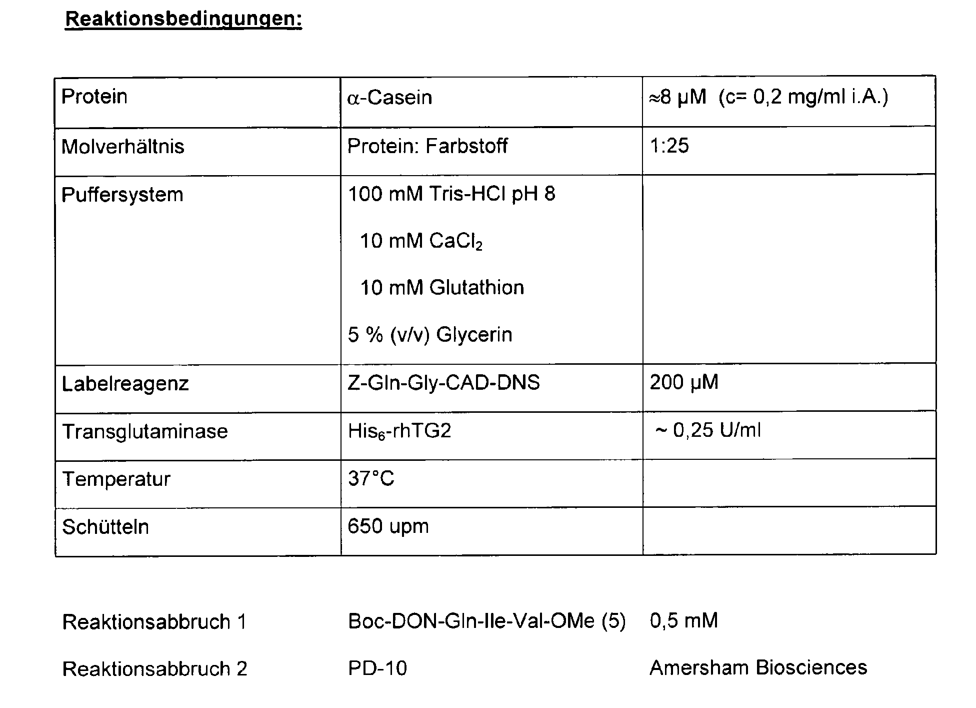

### Versuchsbeschreibung:

Die Durchführung der Reaktionen erfolgt analog der in Beispiel 1 beschriebenen. Nur die Reaktionszeiten und Molaritäten wurden dem verwendeten System angepasst. Im vorliegenden Beispiel wurde α-Casein als Zielprotein eingesetzt. Dieses Protein wird von allen zur Verfügung stehenden Transglutaminasen sowohl als Glutamin- als auch als Lysindonor akzeptiert. Dadurch sind intra- oder intermolekulare Vernetzungsreaktionen möglich. Im gewählten Beispiel wird ein glutaminhaltiges Farbstoffsubstrat eingesetzt. Der Verbleib aktiver Transglutaminase zusammen mit dem Zielprotein im Produkt nach Gelpermationschromatographie ist anhand von hochmolekularen Verunreinigungen deutlich zu erkennen. Das Zielprotein wird weiter modifiziert. Man erhält ohne Einsatz erfindungsgemäßer Inhibitoren ein niederwertiges Produkt. Je nach Art des Einsatzes des markierten Zielproteins kann diese aktive Transglutaminase darüber hinaus störende Effekte bei der Verwendung des Produktes haben.

### Analytik:

| | |
|---|---|
| Polyacrylamidgel | Trenngel 12,5 %ig, Sammelgel 5,0 %ig |
| Puffersystem | Tris-Glycin |
| Auftragsmenge | ~ 1,5 µg / Spur |
| Elektrophorese-System | BioRad Mini-PROTEAN® 3 cell |
| Visualisierung | Videodokumentationsanlage LTF, Fluoreszenzaufnahme UV-Anregung bei 280-350 nm |

Die Fluoreszenzaufnahme des Gels ist in Fig. 3 gezeigt.

### Auswertung:

Die Bildung höhermolekularer Proteinspezies ist in beiden Reaktionsabbruchverfahren erkennbar. Insbesondere nach Abtrennung des ungebundenen Farbstoffes durch Gelpermeationschromatographie tritt dieser Effekt jedoch stark auf. Die Ursache dafür liegt in der Entfernung des Konkurrenzsubstrates (Z-Gln-Gly-CAD-DNS). Die hohe Konzentration dieses Farbstoffsubstrates unterdrückt im eigentlichen Markierungsansatz die Bildung dieser hochmolekularen Proteinspezies dadurch, dass es mit den Glutaminresten des Zielproteins um die Bindungsstelle für Glutamin an der Transglutaminase konkurriert. Entfällt diese Konkurrenz durch Entfernung des ungebundenen Farbstoffsubstrates, so tritt die Quervernetzung in den Vordergrund, da aktive Transglutaminase zusammen mit dem Zielprotein im Produkt zurückbleibt. Die Qualität des Zielproteins nimmt dadurch stark ab. Nach irreversibler Inhibierung der Transglutaminase findet keine weitere Modifizierung des Zielproteins statt.

### BEISPIEL 3

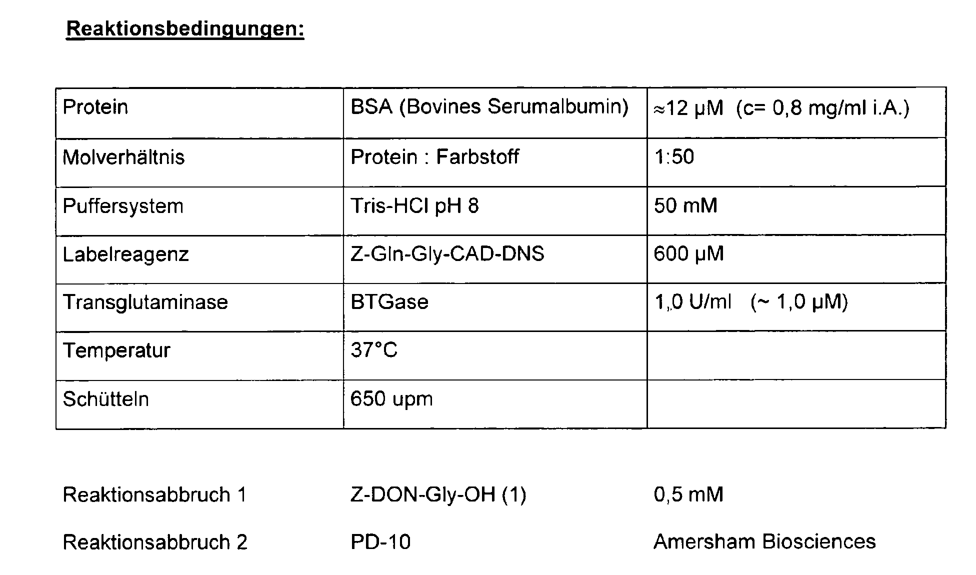

### Versuchsbeschreibung:

In einem von zwei identischen Markierungsansätzen wird nach 5 Minuten die Reaktion durch den Inhibitor Z-DON-Gly-OH abgestoppt (Reaktionsabbruch Nr.1). Direkt nach dem Abstoppen wird eine Probe entnommen und durch SDS-PAGE charakterisiert. Zur Überprüfung der vollständigen und irreversiblen Inhibition wird der Inhibitor durch Gelpermationschromatographie abgetrennt und die Proteinlösung nach erneuter Zugabe von ungebundenem Farbstoff für 30 Min. bei RT aufbewahrt.

Als Kontrollreaktion wird in einem zweiten Ansatz nach 15 Minuten der freie Farbstoff über eine PD-10-Säule abgetrennt (Reaktionsabbruch Nr. 2). Es werden direkt vor und nach der Abtrennung des freien Farbstoffes Proben zur Analyse durch SDS-PAGE entnommen.

### Analytik:

| | |
|---|---|
| Polyacrylamidgel | Trenngel 12,5 %ig, Sammelgel 5,0 %ig |
| Puffersystem | Tris-Glycin |
| Auftragsmenge | ~ 1,5 µg / Spur |
| Elektrophorese-System | BioRad Mini-PROTEAN® 3 cell |
| Visualisierung | Videodokumentationsanlage LTF, Fluoreszenzaufnahme UV-Anregung bei 280-350 nm |

Die Fluoreszenzaufnahme des Gels ist in Fig. 4 gezeigt.

### Auswertung:

Nach Zugabe des Inhibitors wird die Reaktion gestoppt. Auch nach Abtrennung des Inhibitors und 30-minütigem Stehen lassen bei RT in Gegenwart von neu zugesetztem ungebundenen Farbstoff tritt kein weiteres Labeling des Zielproteins auf. Demgegenüber ist bei Reaktionsabbruch durch Gelpermeationschromatographie ein zusätzliches Labeling des Zielproteins erkennbar.

### BEISPIEL 4

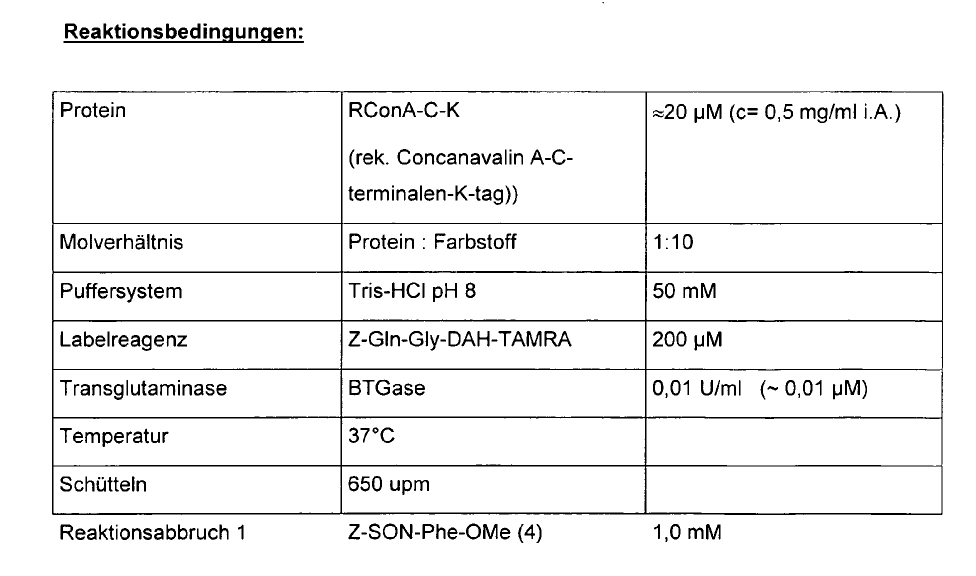

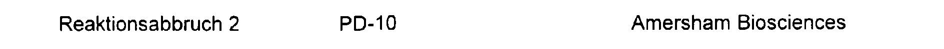

### Versuchsbeschreibung:

In diesem Beispiel wird ein rekombinantes Protein, welches eine zusätzliche Bindungsstelle trägt, die im natürlichen Protein nicht existiert, markiert. Diese Bindungsstelle kann bis zu drei Label tragen und reagiert sehr schnell.

Es werden drei identische Markierungsansätze präpariert. Nach 1 Minute wird in einem Ansatz der freie Farbstoff über eine PD-10-Säule abgetrennt (Reaktionsabbruch Nr. 2) und direkt vor und nach der Abtrennung des freien Farbstoffes SDS-PAGE-Proben zur Überprüfung entnommen.

Im Vergleichsexperiment wird bei zwei Markierungsansätzen nach 2 und 5 Minuten die Reaktion durch Zusatz von Z-SON-Phe-OMe abgestoppt (Reaktionsabbruch Nr. 1). Sofort nach der Inhibierung wird eine SDS-PAGE-Probe entnommen. Zur Überprüfung der vollständigen Inhibierung wird der abgestoppte Markierungsansatz für 30 Min. bei RT aufbewahrt. Anschließend wird eine zweite SDS-PAGE-Probe entnommen.

### Analytik:

| | |
|---|---|
| Polyacrylamidgel | Trenngel 12,5 %ig, Sammelgel 5,0 %ig |
| Puffersystem | Tris-Glycin |
| Auftragsmenge | ~ 1,0 µg / Spur |
| Elektrophorese-System | BioRad Mini-PROTEAN® 3 cell |
| Visualisierung | Videodokumentationsanlage LTF, Fluoreszenzaufnahme UV-Anregung bei 280-350 nm |

Die Fluoreszenzaufnahme des Gels ist in Fig. 5 gezeigt.

### Auswertung:

Es findet nach Inhibitorzugabe keine weitere Markierungsreaktion mehr statt. Bei der Abtrennung des freien Farbstoffes durch eine Chromatographie an einer PD-10-Säule ist eine fortlaufende enzymatische Reaktion erkennbar. Da das rekombinante Concanavalin A durch die Einführung einer spezifischen Transglutaminasebindungsstelle ein sehr gutes Substrat für die bakterielle Transglutaminase ist, verläuft die enzymatische Reaktion trotz sehr geringer Transglutaminaseaktivität im Markierungsansatz sehr schnell. Im vorliegenden Beispiel ist es besonders wichtig, die Transglutaminase sehr schnell und vollständig zu inhibieren. Eine Reaktionssteuerung kann weder durch geringe Transglutaminaseaktivität noch durch eine Begrenzung der Reaktionszeit alleine erreicht werden. Im Fall der Abtrennung des freien Farbstoffes mittels einer PD-10-Säule ist erkennbar, dass die Zeitdauer dieser Reinigungsoperation im Vergleich zur eigentlichen Reaktionsdauer zu lang ist, um ein einheitliches Produkt zu erhalten. Die Reaktionskontrolle muss durch einen sehr schnell wirksamen Inhibitor der Transglutaminase erfolgen.

### BEISPIEL 5

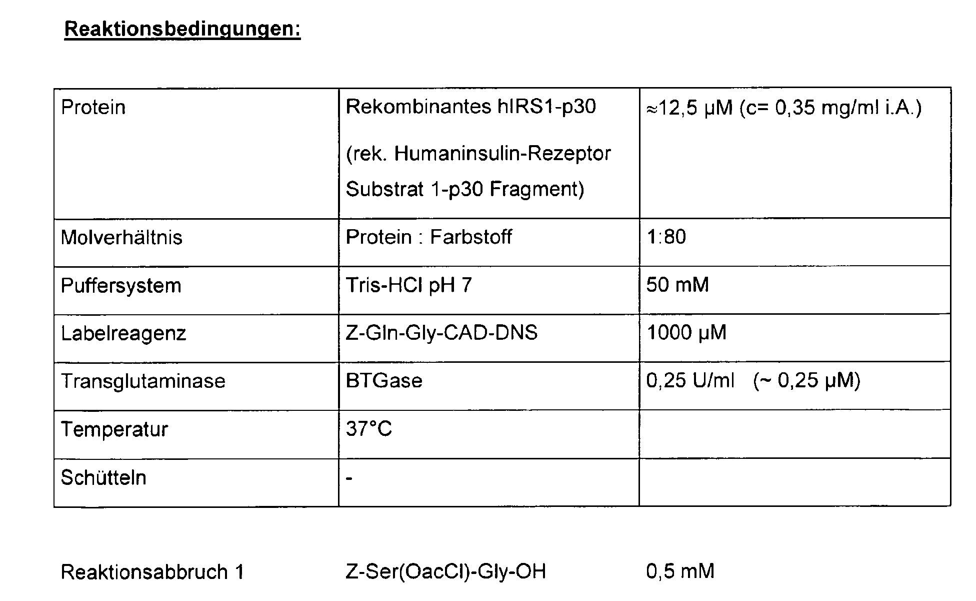

### Versuchsbeschreibung:

In diesem Beispiel eines relevanten Targets der Pharmaforschung können die Vorteile einer Reaktionssteuerung durch selektive, irreversible Transglutaminase-Inhibitoren zusammengefasst werden.

Es werden drei identische Markierungsansätze durchgeführt. Nach jeweils 2, 5 und 10 Minuten werden die Reaktionen durch Zugabe des Inhibitors Z-Ser(OAcCl)-Gly-OH abgestoppt (Reaktionsabbruch Nr. 1). Direkt danach wird jeweils eine Probe entnommen und über SDS-PAGE analysiert. Zum Nachweis der vollständigen Inhibierung wird der abgestoppte Labelansatz für 30 Min. bei RT aufbewahrt. Anschließend wird eine zweite SDS-PAGE-Probe entnommen.

### Analytik:

| | |
|---|---|
| Polyacrylamidgel | Trenngel 12,5%ig, Sammelgel 5,0%ig |
| Puffersystem | Tris-Glycin |
| Auftragsmenge | ~ 2,8 µg / Spur |
| Elektrophorese-System | BioRad Mini-PROTEAN® 3 cell |
| Visualisierung | Videodokumentationsanlage LTF, Fluoreszenzaufnahme UV-Anregung bei 280-350 nm |

Die Fluoreszenzaufnahme des Gels ist in Fig. 6 gezeigt.

### Auswertung:

Durch Abstoppen mit einem Inhibitor bei bestimmten Inkubationszeiten können definierte Markierungsgrade eingestellt werden. Es finden nach Inhibitorzugabe keine weiteren Markierungsreaktionen mehr statt. Wird eine Reaktionszeit von fünf Minuten überschritten, so nimmt der Anteil an fluoreszenzmarkierten Verunreinigungen und höheren Proteinaggregaten, die zusammen mit dem Zielprotein erhalten werden, deutlich zu. Die Qualität des erhaltenen Produktes ist hinsichtlich enthaltener Proteinverunreinigungen im erfindungsgemäßen Verfahren der Prozessführung hoch. Dafür ist es jedoch erforderlich, einen Inhibitor einzusetzen, der schnell und selektiv Transglutaminase inhibiert.

Die folgenden Beispiele zeigen die vollständige und irreversible Inhibierung von Transglutaminasen durch den Einsatz erfindungsgemäßer Inhibitoren.

### BEISPIEL 6

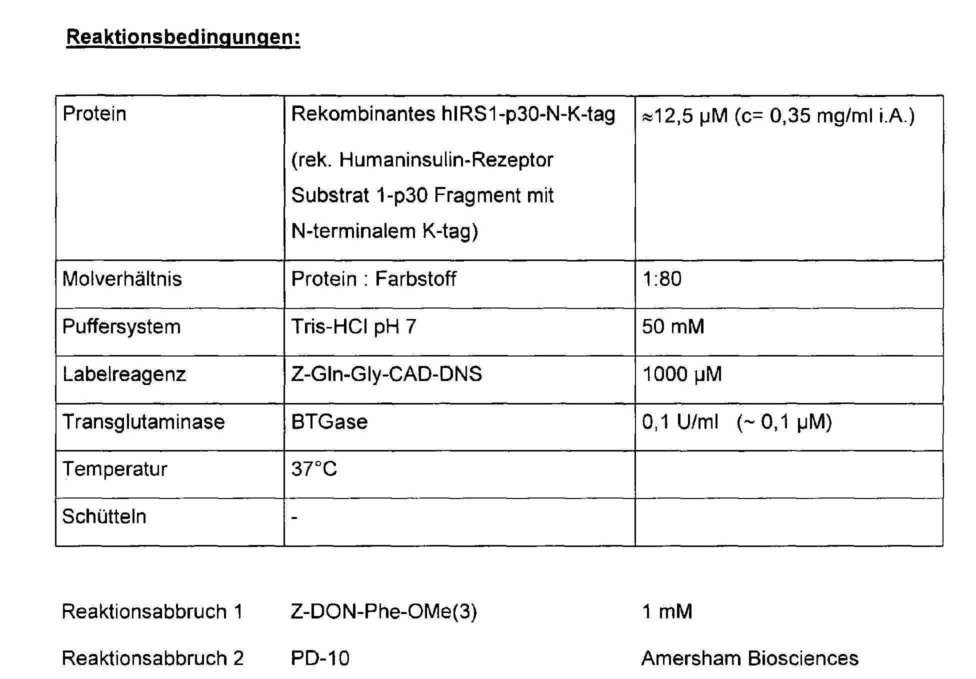

### Versuchsbeschreibung:

In einem von zwei identischen Markierungsansätzen wird nach 5 Minuten die Reaktion mit dem Inhibitor Z-DON-Phe-OMe abgestoppt (Reaktionsabbruch Nr. 1). Direkt nach dem Abstoppen durch Inhibierung wird eine Probe für die SDS-PAGE-Analytik entnommen. Zur Überprüfung der vollständigen Inhibierung werden Farbstoff und Inhibitor durch Gelpermeationschromatographie abgetrennt. Die erhaltene Proteinlösung wird bei RT aufbewahrt, und nach 30 Min., 1 h, 2 h und 4 h werden weitere Proben durch SDS-PAGE charakterisiert.

Als Kontrollexperiment wird nach 5 Minuten der freie Farbstoff über eine PD-10-Säule abgetrennt (Reaktionsabbruch Nr. 2). Charakterisierung durch SDS-PAGE erfolgt direkt nach der Abtrennung des freien Farbstoffes, sowie nach Lagerung bei RT für 30 Min., 1 h, 2 h und 4 h.

### Analytik:

| | |
|---|---|
| Polyacrylamidgel | Trenngel 12,5 %ig, Sammelgel 5,0 %ig |
| Puffersystem | Tris-Glycin |
| Auftragsmenge | ~ 2,8 µg / Spur |
| Elektrophorese-System | BioRad Mini-PROTEAN® 3 cell |
| Visualisierung | Videodokumentationsanlage LTF, Fluoreszenzaufnahme UV-Anregung bei 280-350 nm |

Die Fluoreszenzaufnahme des Gels ist in Fig. 7 gezeigt.

### Auswertung:

Die vollständige und irreversible Inhibition der Transglutaminase kann eindeutig gezeigt werden. Nach Abtrennung von Farbstoff und Inhibitor findet keine weitere Modifikation des Zielproteins statt. Rekombinantes hIRS1-p30-N-K ist sowohl ein K- als auch ein Q-Substrat für die bakterielle Transglutaminase. Daher können zusätzlich zur beabsichtigten Markierungsreaktion Quervernetzungsreaktionen von zwei oder mehreren hIRS1-p30-N-K-Molekülen (intermolekulare Quervernetzung) oder innerhalb eines Moleküls (intramolekulare Vernetzung) unter Transglutaminasekatalyse ablaufen. Verbleibt aktive Transglutaminase zusammen mit dem Zielprotein im Produkt, so wird das zunächst nur wenig verunreinigte Protein mit zunehmender Dauer durch intra- und intermolekulare Vernetzung des Proteins kontaminiert. Solche Modifikationen können erheblichen Einfluss auf die Eigenschaften des Proteins haben.

Die eigentliche Markierungsreaktion kann zwar mit der Abtrennung des freien Farbstoffes abgestoppt werden, aber die Quervernetzung zwischen Proteinmolekülen wird durch aktive Transglutaminase weiter katalysiert. Die Reaktion der Quervernetzung wird bei diesem Protein bevorzugt. Im Verlaufe der eigentlichen Markierungsreaktion kann dies durch ein sehr großes Molverhältnis von Protein zu Farbstoff (1:80) kontrolliert werden. Wird das Konkurrenzsubstrat Z-Gln-Gly-CAD-DNS (durch PD-10-Säule) entfernt, läuft die Reaktion der Quervernetzung im verstärkten Maße ab. Bereits in der Probe direkt nach der Reinigung durch Gelpermeationschromatographie (Spur 2) sieht man, dass ein geringer Anteil von Di-, Tri- und Tetrameren entstanden ist. In den nachfolgenden Proben (Spur 3-6), die bei RT aufbewahrt wurden, wurde das bereits markierte Protein weitgehend intra- und intermolekular vernetzt.

### BEISPIEL 7

### Reaktionsbedingungen:

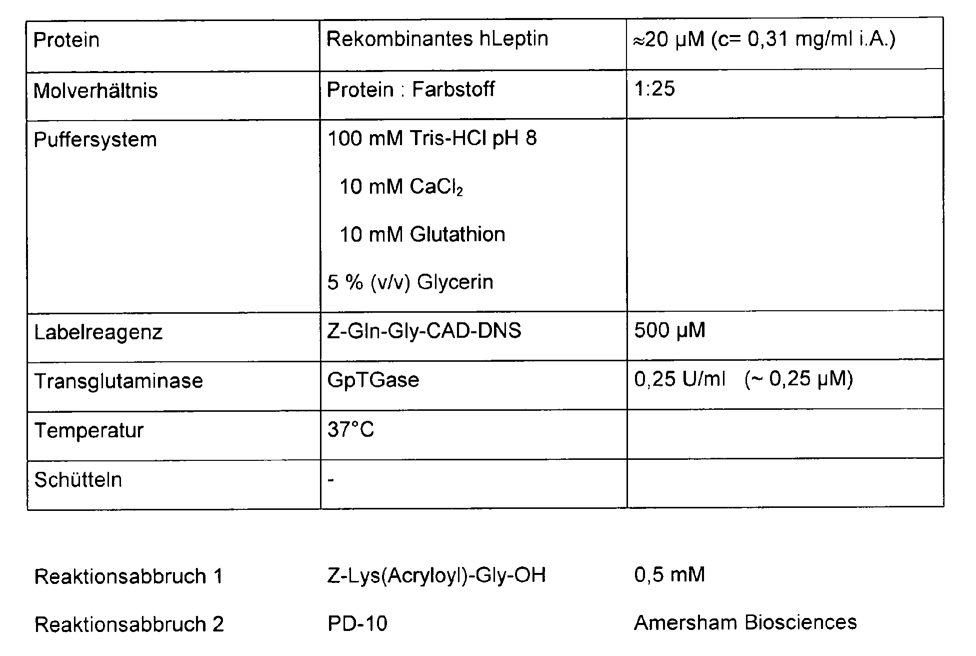

### Versuchsbeschreibung:

Bei einem von zwei identischen Markierungsansätzen wird die Reaktion nach 5 Minuten mit dem Inhibitor Z-Lys(Acryloyl)-Gly-OH abgebrochen. (Reaktionsabbruch Nr. 1). Direkt nach Abstoppen durch Inhibitorzugabe wird eine Probe entnommen und durch RP-HPLC charakterisiert. Zur Überprüfung der vollständigen Inhibierung wird der Markierungsansatz bei RT aufbewahrt. Es werden weitere Proben nach 30 Min. bzw. 60 Min. entnommen und ebenfalls durch RP-HPLC charakterisiert.

Als Kontrollexperiment wird in einem zweiten Ansatz nach 5 Minuten ungebundenes Farbstoffsubstrat über eine PD-10-Säule abgetrennt (Reaktionsabbruch Nr. 2). Es wird direkt nach der Abtrennung des freien Farbstoffes eine Probe zur Reaktionskontrolle durch RP-HPLC entnommen. Die Proteinlösung wird bei RT gelagert. Weitere Proben werden nach 30 Min. bzw. 60 Min. entnommen und charakterisiert. In beiden Verfahrenswegen wird zur Überprüfung der Langzeitstabilität ein Aliquot der erhaltenen Proteinlösung zehn Tage bei -20 °C gelagert und nach dem Auftauen für eine Stunde bei RT gelagert.

### Analytik:

| | | |
|---|---|---|
| **RP-HPLC** | Gerät | Äkta™ basic 10, Amersham Biosciences |
| | Säule | POROS 10R2, 4,6 x 250 mm |
| | Eluent A | 0,1 % (v/v) TFA in bidest.-H₂O |
| | Eluent B | 80 % (v/v) Acetonitril in 0,1 % (v/v) TFA |
| | Flussrate | 2 ml/Min. |
| | Wellenlänge 1 | 214 nm (λₘₐₓ. von Peptidbindungen) |
| | UV-Averaging Time | 0,32 s |
| | Drucklimit | 17 Mpa |
| | Startkonzentration Eluent A | 100 % |
| | Startkonzentration Eluent B | 0 % |
| | Säulenäquilibrierung | 5 CV |
| | Loopspülung | 1,0 ml |
| | Säulenspülung | 1 Min.. |
| | Gradient | 0 - 100% B in 30 Min.. |
| | Säulenreinigung | 10 CV Eluent B (100 %) |
| | Reäquilibrierung | 10 CV Eluent A (100 %) |
| | Probenvolumen | 100 µl |
| | Probenmenge | 26 µg |
| | Probenvorbereitung | 100 µl Probe + 20 µl Eluent B Zentrifugation: 10 Min., RT, 18.000 x g |
| **ESI-MS** | Externe Analytik | MPI, München |

Die Chromatogramme sind in Fig. 8 gezeigt.

### Auswertung:

Z-Lys(Acryloylamid)-Gly-OH inaktiviert Meerschweinchenlebertransglutaminase vollständig. Es finden keine weitere Labeling- und Quervernetzungsreaktionen statt. In dem durch Inhibierung gesteuerten Ansatz fallen nach der Lagerung für 10 Tage bei -20°C geringe Mengen an Protein aus (Chromatogramm Nr. 5). Es finden aber keine weiteren enzymatischen Reaktionen mehr statt.

Bei der Abtrennung des freien Farbstoffes durch eine PD-10-Säule sieht man, dass die katalytische Reaktion nach Abtrennung des Labelreagenzes weiterläuft. Da das rekombinante hLeptin sowohl ein Lysin- als auch ein Glutamin-Substrat für die GpTGase ist, können statt der beabsichtigten Markierungsreaktion noch zusätzlich Quervernetzungen von zwei oder mehreren hLeptin-Molekülen (intermolekulare Quervernetzung) auftreten. Die eigentliche Markierungsreaktion kann zwar durch die Abtrennung des freien Farbstoffes abgestoppt werden, aber die Quervernetzung zwischen Proteinmolekülen findet weiterhin statt. Fällt das Konkurrenzsubstrat Z-Gln-Gly-CAD-DNS (durch PD-10-Säule) weg, läuft die Reaktion der Quervernetzung in verstärktem Maße ab. Es bilden sich hochmolekulare, quervernetzte Proteinaggregate, die während des Zentrifugierens ausfallen. Bei der Probe, die bei -20°C gelagert wurde, ist kein Protein mehr nachweisbar.

### BEISPIEL 8

### Direkter Nachweis der schnellen und vollständigen Inhibition von Transglutaminasen durch den Einsatz erfindungsgemäßer Inhibitoren

Durch den Einsatz der erfindungsgemäßen Inhibitoren müssen die jeweils verwendeten Transglutaminasen sehr schnell und vollständig inhibiert werden. Indirekt kann dies aus der Zusammensetzung der erhaltenen Zielproteine geschlossen werden. Ein direkter Nachweis gelingt durch Online-Messung der Transglutaminaseaktivität. Hierfür wird nach dem Stand der Technik häufig ein Verfahren eingesetzt, bei dem die Steigerung der beobachtbaren Fluoreszenz, durch den Einbau von Monodansylcadaverin in N, N-Dimethylcasein, detektiert wird. (Lorand L., Lockridge M., Campell K., Myhrman R., Bruner-Lorand J., Analytical Biochemistry, *44*, 1971, 221-231). Die durch den Einbau in das Protein beobachtbare Fluoreszenzsteigerung endet, sobald Transglutaminase kein weiteres Monodansylcadaverin in N, N-Dimethylcasein einbaut. Dieser Aktivitätsassay wurde in der Literatur beschrieben, um Inhibitoren von Transglutaminase zu charakterisieren (z. B. Pliura, D. H., Bonaventura B.J., Pauls H.W., Killackey J.F., Krantz A.; J. Enzyme Inhibition; 6, 1992, 181-194).

### Durchführung:

### Puffer A

50 mM Tris/HCl, pH 7, 10 mM Glutathion, 25 µM Monodansylcadaverin, 2,5 % DMSO, 7 µM N,N-Dimethylcasein, 10 mM CaCl₂, 5 % Glycerin
Stammlösung Inhibitoren: 5 mM in 20% DMSO / 80% Puffer
Enzymlösung: 0,9 mg/ml Transglutaminase in 1 ml Puffer B mit folgender Zusammensetzung (50 mM Tris/HCl, pH 7, 10 mM Glutathion, 10 mM CaCl₂, 5 % Glycerin)
Fluoreszenzspektrophotometer: Cary Eclipse, Varian; Software: Cary Eclipse,
Excitationswellenlänge: 332 nm, Emissionswellenlänge: 500 nm, Detektorverstärkung: 600 Volt

1060 µl Puffer A werden auf 37°C temperiert. Zu dieser Lösung werden 20 µl der Enzymlösung in Puffer B zugesetzt und der Anstieg der Fluoreszenz beobachtet. In einer Vergleichsprobe wird der lineare Anstieg der Fluoreszenz über mindestens 20 Minuten nach Zusatz von 20 µl Puffer B (ohne Inhibitor) gezeigt. In den Messungen werden nach jeweils drei Minuten linearen Anstieges 120 µl Inhibitorstammlösung zugesetzt (Konzentration des Inhibitors im Ansatz: 500 µM). Zu beobachten ist der sofortige Abbruch des Fluoreszenzanstieges nach Zugabe des Inhibitors.

Die Fluoreszenzspektrogramme sind in Fig. 9 gezeigt.

Die folgenden Beispiele zeigen die Substratspezifität verschiedener Transglutaminasen.

### BEISPIEL 9

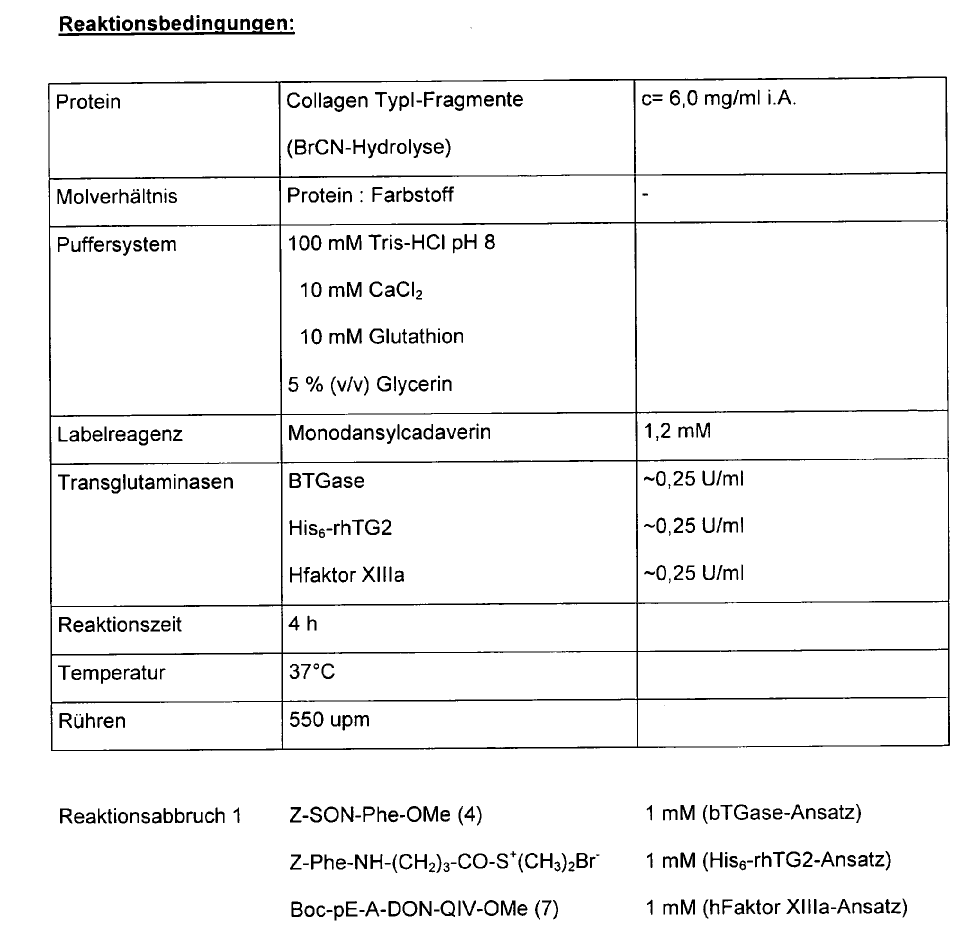

### Versuchsbeschreibung:

Drei Markierungsansätze mit drei verschiedenen Transglutaminasen werden unter ansonsten identischen Bedingungen durchgeführt. Nach 4 Stunden wird bei jedem Markierungsansatz die Reaktion mit dem entsprechenden Inhibitor abgestoppt (Reaktionsabbruch Nr. 1). Direkt nach der Inhibition wird eine Probe entnommen und durch SDS-PAGE charakterisiert.

### Analytik:

| | |
|---|---|
| Polyacrylamidgel | Trenngel 12,5 %ig, Sammelgel 5,0 %ig |
| Puffersystem | Tris-Glycin |
| Auftragsmenge | ~ 50 µg / Spur |
| Elektrophorese-System | BioRad Mini-PROTEAN® 3 cell |
| Visualisierung | Videodokumentationsanlage LTF, Fluoreszenzaufnahme UV-Anregung bei 280-350 nm |

Die Fluoreszenzaufnahme des Gels ist in Fig. 10 gezeigt.

### Auswertung:

Die Substratspezifitäten der verschiedenen Transglutaminasen können anhand der unterschiedlichen Polypeptidfragmente, die als Substrat erkannt wurden, nachgewiesen werden. In diesem Beispiel werden die Glutaminreste an den Peptiden markiert. Das fluoreszierende Bandenmuster ist deutlich abweichend. His₆-rhTG2 bzw. hFaktor Xllla akzeptieren hauptsächlich zwei Fragmente (His₆-rhTG2: Spur 1, Doppelbande unterhalb 34 kDa; hFaktor XIIIa: Spur 2, 34 und 19 kD). Alle anderen Collagenfragmente werden nur sehr schwach oder gar nicht markiert. Demgegenüber ist eine solche bevorzugte Markierung eines Fragments gegenüber den anderen durch die bakterielle TGase (Spur 4) nicht erkennbar. Alle 7 Fragmente zwischen 19 und 59 kDa zeigen in etwa die gleiche Fluoreszenzintensität.

Diese unterschiedliche Spezifität verschiedener Transglutaminasen ermöglicht es, den Reaktionsverlauf durch gezielte Auswahl der am besten geeigneten Transglutaminase derart zu steuern, dass das Zielprotein nur an wenigen Stellen markiert wird.

### BEISPIEL 10

### Reaktionsbedingungen:

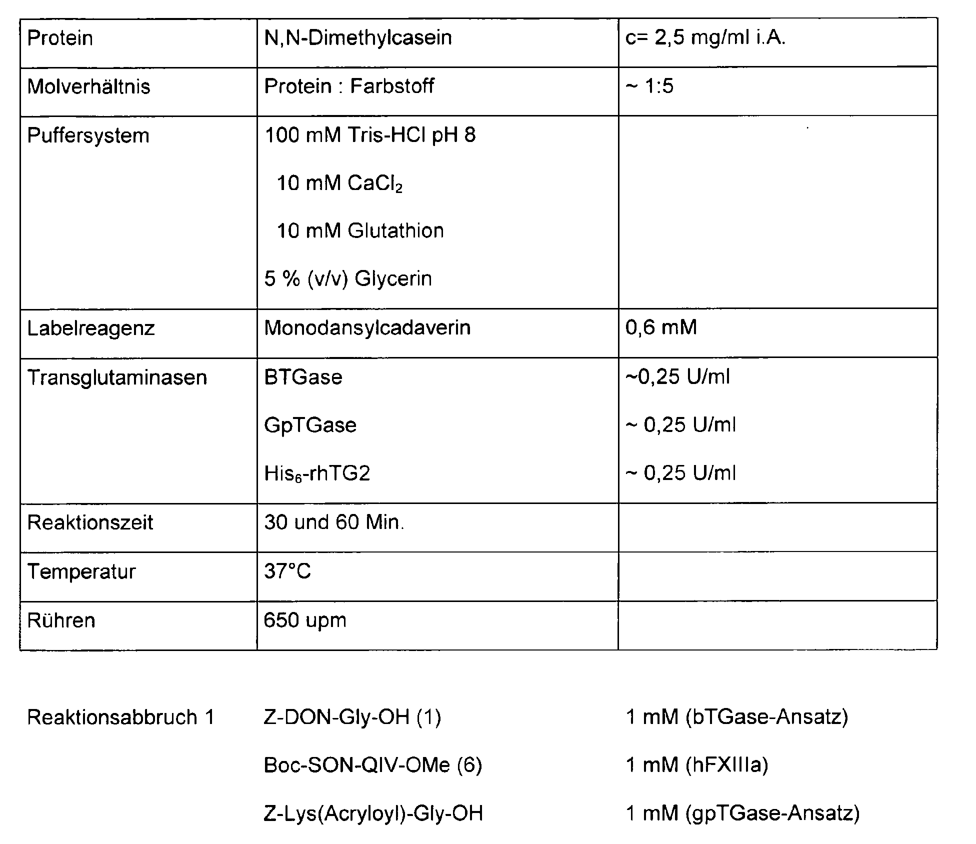

### Versuchsbeschreibung:

Von den drei Labelansätzen mit den verschiedenen Transglutaminasen werden jeweils nach 30 und 60 Minuten Proben entnommen und die Reaktion mit dem entsprechenden Inhibitor abgestoppt (Reaktionsabbruch Nr. 1). Direkt nach dem Reaktionsabbruch wird eine Probe für SDS-PAGE-Analytik entnommen.

### Analytik:

| | |
|---|---|
| Polyacrylamidgel | Trenngel 12,5 %ig, Sammelgel 5,0 %ig |
| Puffersystem | Tris-Glycin |
| Auftragsmenge | ~ 20 µg / Spur |
| Elektrophorese-System | BioRad Mini-PROTEAN® 3 cell |
| Visualisierung | Videodokumentationsanlage LTF, Fluoreszenzaufnahme UV-Anregung bei 280-350 nm |

Die Fluoreszenzaufnahme des Gels ist in Fig. 11 gezeigt.

### Auswertung:

Wie auch in Beispiel 9 werden Glutaminreste des Proteins markiert. Die Substratspezifitäten zwischen den verschiedenen Transglutaminasen sind bei N,N-Dimethylcasein aber noch stärker ausgeprägt. Während bTGase relativ unspezifisch und mehrfach reagiert, ist die Substratspezifität im Fall der Meerschweinchenlebertransglutaminase höher. Es werden weniger Dansylmoleküle in das Protein eingebaut, und die im kommerziell erhältlichen N,N-Dimethylcasein enthaltenen Verunreinigungen werden nur in untergeordnetem Maß als Substrat erkannt. Humaner Blutgerinnungsfaktor Xllla erkennt N,N-Dimethylcasein als Substrat. Durch die geringere Affinität wird Casein unter ansonsten identischen Bedingungen deutlich geringer markiert. Fragmente bzw. andere Verunreinigungen werden durch FXIIIₐ nicht markiert.

### BEISPIEL 11

### Reaktionsbedingungen:

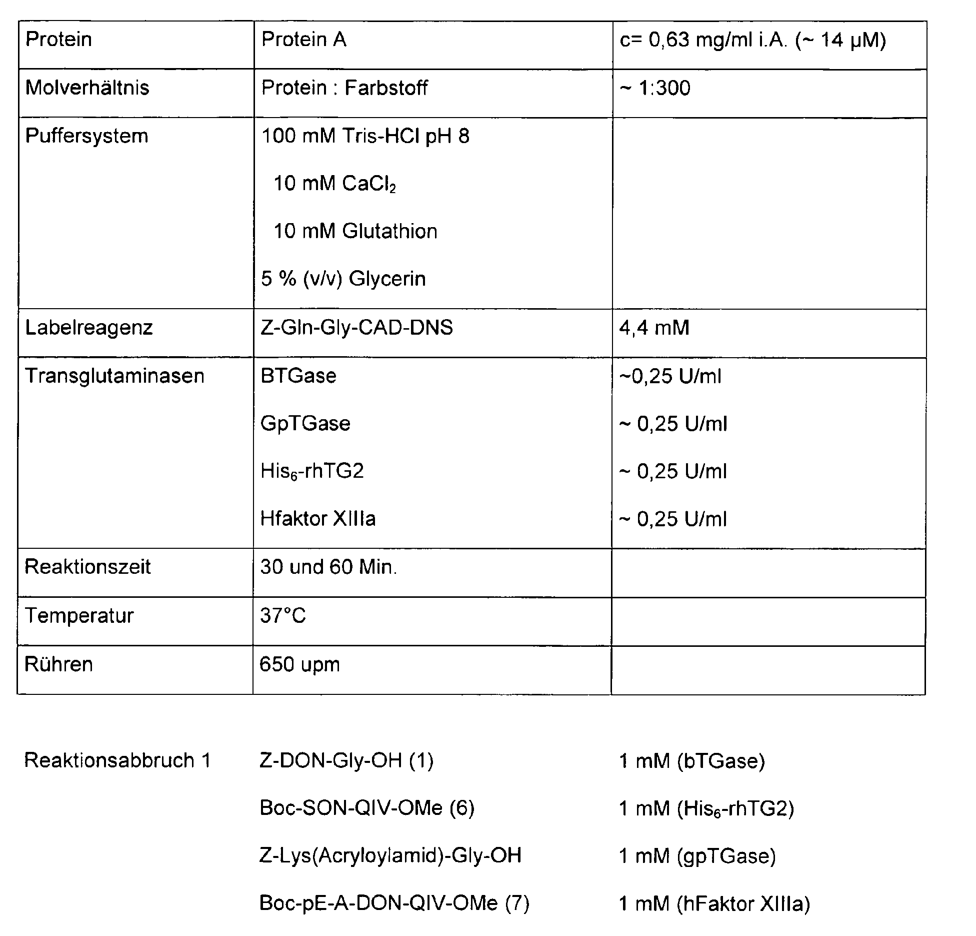

### Versuchsbeschreibung:

Es werden vier Labelansätze mit den verschiedenen Transglutaminasen präpariert. Nach 4 Stunden werden die Reaktionen mit den entsprechenden Inhibitoren abgestoppt (Reaktionsabbruch Nr.1, s.o.). Direkt nach dem Abstoppen mittels Inhibierung wird eine SDS-PAGE-Probe entnommen.

### Analytik:

| | |
|---|---|
| Polyacrylamidgel | Trenngel 12,5 %ig, Sammelgel 5,0 %ig |
| Puffersystem | Tris-Glycin |
| Auftragsmenge | ~ 3 µg / Spur |
| Elektrophorese-System | BioRad Mini-PROTEAN® 3 cell |
| Visualisierung | Videodokumentationsanlage LTF, Fluoreszenzaufnahme UV-Anregung bei 280-350 nm |

Die Fluoreszenzaufnahme des Gels ist in Fig. 12 gezeigt.

### Auswertung:

Deutlich zu erkennen ist, dass das fluoreszierende Bandenmuster sehr unterschiedlich ist. Quervernetzung und Labeling sind stets Konkurrenzreaktionen, wenn das Zielprotein, wie im vorliegenden Fall, sowohl Glutamin- als auch Amin-Donor für die eingesetzte Transglutaminase ist. Intermolekulare Quervernetzungsreaktionen führen zur Ausbildung hochmolekularer Aggregate. Diese sind unter Katalyse mit bTGase dominierend. Unter dem katalytischen Einfluss von hFXllla sind keine solchen Quervernetzungen detektierbar. Das bedeutet, hfaktor Xllla akzeptiert unter diesen Reaktionsbedingungen keine Glutamine von Protein A. His₆-rhTG2-MalE und GpTGase liegen in den Substratspezifitäten dazwischen.

Da viele Proteine von sehr substratspezifischen Transglutaminasen nicht erkannt werden, kann in diesen Fällen durch den Einsatz weniger spezifischer Transglutaminasen ein selektives Reaktionsmuster erreicht werden.

## Patentansprüche

1. Verfahren zur Transglutaminase-katalysierten spezifischen Proteinmodifizierung, welches durch mindestens einen selektiven irreversiblen Transglutaminase-Inhibitor gesteuert wird.

2. Verfahren nach Anspruch 1, **dadurch gekennzeichnet, dass** die als Katalysator eingesetzte Transglutaminase durch die Zugabe von mindestens einem selektiven irreversiblen Transglutaminase-Inhibitor schnell und vollständig inhibiert wird.

3. Verfahren nach Anspruch 1 oder 2, **dadurch gekennzeichnet, dass** mehrere Proteinmodifizierungsreaktionen, welche durch Transglutaminasen mit unterschiedlicher Substratspezifität katalysiert werden, nacheinander oder gleichzeitig durchgeführt werden.

4. Verfahren nach einem der Ansprüche 1-3, **dadurch gekennzeichnet, dass** es sich bei der Proteinmodifizierung um eine Markierung handelt.

5. Verfahren nach Anspruch 4, **dadurch gekennzeichnet, dass** es sich bei der Markierung um eine Markierung mit einem Farbstoff, Fluoreszenzlabel, Chemolumineszenzlabel, radioaktiven Label, Enzymlabel, Redoxlabel, Immunlabel, Biotin-Label, Spinlabel handelt.

6. Verfahren nach einem der Ansprüche 1-3, **dadurch gekennzeichnet, dass** die Proteinmodifizierung die Kopplung des Proteins mit einer nicht-markierten reaktiven oder stabilisierenden Gruppierung umfaßt.

7. Verfahren nach Anspruch 6, **dadurch gekennzeichnet, dass** die stabilisierende Gruppierung aus PEG und Derivaten davon, Dextran und Derivaten davon sowie Stärke und Stärkederivaten ausgewählt ist.

8. Verfahren nach einem der Ansprüche 1-7, **dadurch gekennzeichnet, dass** der oder die Transglutaminase-Inhibitor(en) aus der Gruppe von Verbindungen der Formel I 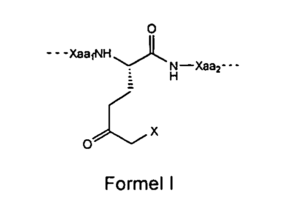 worin X N₂, S⁺(CH₃)₂, RCOO oder R-S ist, Xaa₁ und Xaa₂, die gleich oder verschieden sein können, jeweils eine Aminoschutzgruppe, eine Aminosäure oder ein Aminosäurederivat oder eine Peptidylgruppierung mit 2-8, vorzugsweise 6-8, Aminosäureresten oder Derivaten von Aminosäuren darstellen,
oder aus N^{α}-Benzyloxycarbonyl-L-serinyl-(O-chloracetyl)glycin, N^{α}-Benzyloxycarbonyl-L-lysinyl(ε-acryloyl)glycin und N-(N°-Benzyloxycarbonyl-L-phenylalaninyl)-(4-amino-2-oxo-dimethylsulfonium)pentan-Salz ausgewählt ist bzw. sind.

9. Verfahren nach Anspruch 8, **dadurch gekenzeichnet, daß** die Aminosäurederivate eine N-terminale tert.-Butyloxycarbonyl- oder Benzyloxycarbonylschutzgruppe umfassen.

10. Verfahren nach Anspruch 8 oder 9, **dadurch gekennzeichnet, dass** der oder die Transglutaminase-Inhibitor(en) aus N^{α}-Benzyloxycarbonyl-L-(6-diazo-5-oxonorleucinyl)glycin, N^{α}-Benzyloxycarbonyl-L-(6-dimethylsulfonium-5-oxonorleucinyl)glycinmethylester, N^{α}-Benzyloxycarbonyl-L-(6-diazo-5-oxonorleucinyl)phenylalaninmethylester, N^{α}-Benzyloxycarbonyl-L-(6-dimethylsulfonium-5-oxonorleucinyl)phenylalaninmethylester, N^{α}-tert.-Butyloxycarbonyl-L-(6-diazo-5-oxonorleucinyl)-glutaminyl-L-isoleucinyl-L-valinmethylester, N^{α}-tert.-Butyloxycarbonyl-L-pyroglutaminyl-L-alaninyl-L-(6-diazo-5-oxonorleucinyl)-L-glutaminyl-L-isoleucinyl-L-valinmethylester, N^{α}-tert.-Butyloxycarbonyl-L-(6-dimethylsulfonium-5-oxonorleucinyl)-L-glutaminyl-L-isoleucinyl-L-valinmethylester, N^{α}-Benzyloxycarbonyl-L-serinyl(O-chloracetyl)glycin, N^{α}-Benzyloxycarbonyl-L-lysinyl(ε-acryloyl)glycin und N-(N^{α}-Benzyloxycarbonyl-L-phenylalaninyl)-(4-amino-2-oxo-dimethylsulfonium)pentan-Salz ausgewählt ist bzw. sind.

11. Verfahren nach einem der Ansprüche 1-9, **dadurch gekennzeichnet, dass** die Transglutaminase(n) aus bakteriellen Transglutaminasen und Vertebraten-Glutamiasen, insbesondere Säuger-Transglutaminasen, ausgewählt ist bzw. sind.

12. Verfahren nach Anspruch 11, **dadurch gekennzeichnet, dass** die Transglutaminase(n) aus bakterieller Transglutaminase aus *Streptomyces mobaraensis* (E.C: 2.3.313), humaner Transglutaminase 2, humanem Blutgerinnungsfaktor XIII und Meerschweinchenlebertransglutaminase ausgewählt ist bzw. sind.

13. Selektive irreversible Transglutaminase-Inhibitoren der Formel (I), 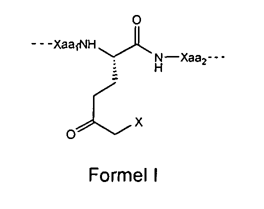 worin X N₂, S⁺(CH₃)₂, RCOO oder R-S ist, Xaa₁ und Xaa₂, die gleich oder verschieden sein können, jeweils eine Aminoschutzgruppe, eine Aminosäure oder ein Aminosäurederivat oder eine Peptidylgruppierung mit 2-8, vorzugsweise 6-8, Aminosäureresten oder Derivaten von Aminosäuren darstellen, mit der Maßgabe, dass der Inhibitor nicht Ac-Prolinyl-glutaminyl-prolinyl-(6-diazo-5-oxonorleucinyl)-leucinyl-prolinyl-phenylalaninyl-NH₂ ist.

14. Selektive irreversible Transglutaminase-Inhibitoren nach Anspruch 13, **dadurch gekennzeichnet, dass** die Aminosäurederivate eine N-terminale tert.-Butyloxycarbonyl- oder Benzyloxycarbonylschutzgruppe umfassen.

15. Verfahren zur Herstellung von seitenkettenmodifizierten Peptiden als selektive irreversible Transglutaminase-Inhibitoren, **dadurch gekennzeichnet, dass** in einem als Transglutaminasesubstrat bekanntem Peptid das Substratglutamin durch eine in Gammaposition in ein Methylketon umgewandelte Glutaminsäure ersetzt wird.

16. Verfahren nach Anspruch 15, **dadurch gekennzeichnet, dass**
a) bei einem Ausgangspeptid, in dem die Gammaposition der Ziel-Glutaminsäure ungeschützt vorliegt, diese Carbonsäure als Mischanhydrid mit, vorzugsweise, Isobutylchloroformiat aktiviert wird,
b) durch Zusatz von Diazomethan diese aktivierte Carbonsäure in ein Methylketon überführt wird,
c) gegebenenfalls aus diesem Diazomethylketon, welches einen ersten selektiven irreversiblen Transglutaminase-Inhibitor darstellt, durch Umsetzung mit Bromwasserstoffsäure in Essigsäure ein reaktives Brommethylketon-Intermediat hergestellt wird und
d) die Bromgruppierung durch Carboxylate, Thiole oder andere Anionen substituiert wird, wodurch weitere selektive irreversible Transglutaminase-Inhibitoren resultieren.
